# EUROPEAN PATENT APPLICATION

(11) **EP 4 599 876 A1**
(43) Date of publication of application: **13.08.2025**
(21) Application number: 25182891.9
(22) Date of filing: 20.08.2021
(51) Int. Cl.: A61M 16/12, A61M 16/20, A61M 16/10, A61M 16/00, A61K 33/00, A61P 11/00, A61P 9/00

(54) **GENERATION OF NITRIC OXIDE AND DELIVERY SYSTEMS**

(30) Priority: 20.08.2020 US 202063067934 P; 31.08.2020 US 202063072600 P; 26.01.2021 US 202163141657 P
(62) Divisional of application: 21859311.9
(71) Applicant: The UAB Research Foundation, Birmingham AL 35294 (US); The Johns Hopkins University, Baltimore, MD 21218 (US)
(72) Inventor: ENIKA, Nagababu, Birmingham, Alabama, 35243 (US); BERKOWITZ, Dan E., Mountain Brook, Alabama, 35223 (US)
(74) Representative: Berggren Oy

(57) **Abstract**

Provided herein are NO generating systems, methods for generating NO for inhaled therapy, and methods of treating patients in need of inhaled NO. The systems can include a reaction vessel having a headspace for NO accumulation, a NO storage system, and a gas mixing chamber. NO gas generated in the reaction vessel can be delivered to the NO storage system via pressure generated by generation of the NO gas, then the NO gas is mixed with a carrier gas in a gas mixing chamber. NO can be generated at the point of patient delivery.

## Description

### CROSS-REFERENCE TO RELATED APPLICATIONS

This application claims the benefit of and priority to U.S. Provisional Application Serial No. 63/067,934, having the title "GENERATION OF NITRIC OXIDE AND DELIVERY SYSTEMS", filed on August 20, 2020; U.S. Provisional Application Serial No. 63/072,600, having the title "GENERATION OF NITRIC OXIDE AND DELIVERY SYSTEMS", filed on August 31, 2020; and U.S. Provisional Application Serial No. 63/141,657, having the title "GENERATION OF NITRIC OXIDE AND DELIVERY SYSTEMS", filed on January 26, 2021; the disclosures of each of which are incorporated herein by reference in their entireties.

### BACKGROUND

Pulmonary hypertension (PH) is caused by pulmonary arterial diseases, lung diseases (e.g., acute respiratory distress syndrome, chronic obstructive pulmonary disease, chronic venous pulmonary thromboembolic disease, sleep apnea, and high-altitude exposure), infections, and intra- and postoperative cardiac surgeries. Mortality occurs mostly by right heart right ventricular (RV) failure at the age adjusted rate of 4 to 12.3 per 100,000 in the US, despite the use of costly pharmacologic treatments (e.g., sildenafil, prostacyclin analogs, and endothelin inhibitors). Inhaled nitric oxide (iNO) therapy is the gold standard for treating pulmonary-specific vasodilation without compromising cardiac index and systemic blood pressure. Current iNO therapy requires a complex and expensive (approximately $180/hour) system of gaseous NO storage cylinders, transportation, and devices to monitor and regulate the dilution and delivery of nitric oxide (NO), O₂, and nitrogen dioxide (NO₂). Due to the complexity and expense, this therapy is available only in the intensive care units and operating rooms of established hospitals.

### SUMMARY

Embodiments of the present disclosure provide for NO generating systems, NO generating and delivery systems, methods for generating NO, methods of treating a patient in need of inhaled NO, and the like.

An embodiment of the present disclosure includes a NO generating system that can include a reaction vessel having a headspace for NO accumulation, a NO storage system, and a gas mixing chamber. The NO gas generated in the reaction vessel can be delivered to the NO storage system via pressure generated by generation of the NO gas, then the NO gas can be mixed with a carrier gas in the gas mixing chamber. The gas mixing chamber can include a valve, a nozzle, a carrier gas inlet, and an outlet for a breathing tube, where the valve and nozzle control a flow rate of NO to the gas mixing chamber.

An embodiment of the present disclosure also includes a method for generating NO for inhaled therapy. The method can include reacting a nitrite and an electron donor compound in an aqueous solution. The reaction can be performed under anaerobic conditions at a pH of about 3 to 3.5. The method can also include reacting a nitrite and a thiol (RSH) in an aqueous solution. The reaction can be performed under anaerobic conditions in the absence of light at a first pH of about 1 to 4.5, wherein the reaction produces RSNO and at least a portion of the RSNO decomposes to NO. The nitrite can be selected from the group consisting of sodium nitrite, potassium nitrite calcium nitrite, zinc nitrite, copper nitrite, rubidium nitrite, strontium nitrite, and barium nitrite. The RSNO can be rapidly decomposed to NO by a metal catalyst to generate a bulk amount of NO. The metal catalyst can be cuprous chloride.

An embodiment of the present disclosure also includes a NO generating system. The system can include a reaction vessel having a headspace for NO accumulation, a NO storage system, and a gas mixing chamber. NO gas can be generated in the reaction vessel and delivered to the NO storage system via pressure generated by generation of the NO gas, then the NO gas can be mixed with a carrier gas in the gas mixing chamber.

An embodiment of the present disclosure also includes a method of treating a patient in need of inhaled NO. The method can include reacting a nitrite and an electron donor compound in a reaction vessel in an aqueous solution. The reaction can be performed under anaerobic conditions at a pH of about 3 to 5 to form NO. The NO can be removed from the vessel to a NO storage system. The NO can be diluted to 80 ppm or less with oxygen in a mixing chamber. The diluted NO can then be provided to the patient for inhalation via a tube.

Other compositions, apparatus, methods, features, and advantages will be or become apparent to one with skill in the art upon examination of the following drawings and detailed description. It is intended that all such additional compositions, apparatus, methods, features and advantages be included within this description, be within the scope of the present disclosure, and be protected by the accompanying claims.

### BRIEF DESCRIPTION OF THE DRAWINGS

Further aspects of the present disclosure will be more readily appreciated upon review of the detailed description of its various embodiments, described below, when taken in conjunction with the accompanying drawings.
Figure 1 is a schematic illustration of the generation of NO (part 1), the storage and dispensation of NO (part 2), the mixing of the NO with oxygen (part 3), and the delivery/monitoring of the therapeutic gas (part 4) in accordance with embodiments of the present disclosure.
Figure 2 is a schematic illustration of an example reaction vessel design to generate NO under strict anaerobic conditions.
Figure 3 is a schematic illustration of an example mixing chamber in accordance with embodiments of the present disclosure.
Figure 4 is a schematic illustration of an example nozzle that is used to increase the flow velocity of NO in accordance with embodiments of the present disclosure.
Figure 5A is a schematic illustration of an example NO-generating device in accordance with embodiments of the present disclosure. Figure 5B is a camera image showing a nitric oxide and a nitrogen dioxide dual gas electrochemical sensor in accordance with embodiments of the present disclosure.
Figures 6A-6D illustrate NO synthesis by reduction of sodium nitrite with ascorbic acid under acidic conditions in accordance with embodiments of the present disclosure. Figure 6A shows the percent nitrite reduction in the presence of increasing concentrations of ascorbic acid in distilled water. Figure 6B shows the conversion of nitrite to NO by ascorbic acid in the presence of additional acid in the reaction mixture. Figure 6C illustrates conversion of nitrite to NO in acetate buffer media. Figure 6D shows that the maximum amount of nitrite is reduced to NO in 1 M acetate buffer at pH 3.5.
Figures 7A-7C show S-nitrosothiols as a source of NO conditions in accordance with embodiments of the present disclosure. Figure 7A shows the generation and decomposition of SNOAC. Figure 7B shows the release of NO from SNOAC. Figure 7C depicts bulk NO gas generation from SNOAC.
Figure 8 is a schematic illustration of an example NO-generating device capable of NO delivery using regulation of gas directly from the reaction vessel in accordance with embodiments of the present disclosure.
Figure 9A is a schematic illustration of an example portable NO-generating and delivery device in accordance with embodiments of the present disclosure. Figures 9B, 9C, and 9D are examples of mixing chambers for use with an NO-generating device in accordance with embodiments of the present disclosure.
Figure 10 provides schemes showing that RSNO spontaneously decays via homolytic cleavage to generate RS^{•} and NO^{•} or heterolytic cleavage to RS⁻ and NO⁺ and Mechanism for sustained release of nitric oxide (NO) gas, respectively. RS*, Thiyl radical; NO, Nitric oxide; RS ⁻,Thiyl anion; NO⁺,Nitrosonium ion; RSH, thiol.
Figure 11 shows a mechanism for sustained release of nitric oxide (NO) gas in accordance with embodiments of the present disclosure. RSH, N-acetylcysteine; RSNO, S-nitrosoacetylcysteine; RS*, Thiyl radical; RSSR, disulfide; ROH, antioxidant; RO, antioxidant quinone.
Figure 12 shows an example of a vessel for performing chemical reactions to generate NO under anaerobic conditions and from which the NO can be extracted in accordance with embodiments of the present disclosure.
Figure 13 shows the effect of pH on the release of NO from RSNO in accordance with embodiments of the present disclosure.
Figures 14A and 14B are graphs showing NO release during the reaction of RSH with nitrite: NAC was reacted with nitrite in 0.25 M HCl to generate RSNO. Then, RSNO decay (Figure 14A) and NO release (Figure 14B) were measured.
Figures 15A and 15B are graphs showing sustained release of NO for more than 1 hr in accordance with embodiments of the present disclosure. About 90% of nitrite was converted to RSNO and maintained a steady state level for 6 hrs and then slowly weaned off to 80% in 15 hrs (Figure 15A). The unreacted nitrite reacts slowly with RSH at less acidic pH of 4.5 to generate RSNO to compensate for the decaying RSNO. NO release also followed the same trend (Figure 15B)
Figures 16A and 16B are graphs showing sustained release of NO for more than 6 hrs in accordance with embodiments of the present disclosure. About 90% of nitrite was converted to RSNO, which maintained a steady state level for 10 h and then slowly decreased to 80% in 15 h (Figure 16A). The unreacted nitrite reacts slowly with RSH at the less acidic pH of 4.8 to generate RSNO to compensate for the decaying RSNO. NO release followed a similar trend to that of RSNO decay (Figure 16B).
Figures 17A and 17B are graphs showing sustained release of NO for more than 10 hrs. in accordance with embodiments of the present disclosure. The steady state level of SNOAC was maintained for 15 h and later declined by approximately 20% at 24 h.
Figures 18 is an example of portable device for NO generation in accordance with embodiments of the present disclosure.
Figures 19 and 20 are example setups for benchtop devices for NO generation in accordance with embodiments of the present disclosure.

The drawings illustrate only example embodiments and are therefore not to be considered limiting of the scope described herein, as other equally effective embodiments are within the scope and spirit of this disclosure. The elements and features shown in the drawings are not necessarily drawn to scale, emphasis instead being placed upon clearly illustrating the principles of the embodiments. Additionally, certain dimensions may be exaggerated to help visually convey certain principles. In the drawings, similar reference numerals between figures designate like or corresponding, but not necessarily the same, elements.

### DETAILED DESCRIPTION

Before the present disclosure is described in greater detail, it is to be understood that this disclosure is not limited to particular embodiments described, and as such may, of course, vary. It is also to be understood that the terminology used herein is for the purpose of describing particular embodiments only, and is not intended to be limiting, since the scope of the present disclosure will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure. The upper and lower limits of these smaller ranges may independently be included in the smaller ranges and are also encompassed within the disclosure, subject to any specifically excluded limit in the stated range. Where the stated range includes one or both of the limits, ranges excluding either or both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

As will be apparent to those of skill in the art upon reading this disclosure, each of the individual embodiments described and illustrated herein has discrete components and features which may be readily separated from or combined with the features of any of the other several embodiments without departing from the scope or spirit of the present disclosure. Any recited method can be carried out in the order of events recited or in any other order that is logically possible.

Embodiments of the present disclosure will employ, unless otherwise indicated, techniques of anesthesiology, molecular medicine, chemistry, material science, and the like, which are within the skill of the art.

The following examples are put forth so as to provide those of ordinary skill in the art with a complete disclosure and description of how to perform the methods and use the devices and compositions disclosed and claimed herein. Efforts have been made to ensure accuracy with respect to numbers (e.g., amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible.

It must be noted that, as used in the specification and the appended claims, the singular forms "a," "an," and "the" include plural referents unless the context clearly dictates otherwise.

As used herein, the following terms have the meanings ascribed to them unless specified otherwise. In this disclosure, "consisting essentially of" or "consists essentially" or the like, when applied to methods and compositions encompassed by the present disclosure refers to compositions like those disclosed herein, but which may contain additional structural groups, composition components or method steps (or analogs or derivatives thereof as discussed above). Such additional structural groups, composition components or method steps, etc., however, do not materially affect the basic and novel characteristic(s) of the compositions or methods, compared to those of the corresponding compositions or methods disclosed herein. "Consisting essentially of" or "consists essentially" or the like, when applied to methods and compositions encompassed by the present disclosure have the meaning ascribed in U.S. Patent law and the term is open-ended, allowing for the presence of more than that which is recited so long as basic or novel characteristics of that which is recited is not changed by the presence of more than that which is recited, but excludes prior art embodiments.

### Definitions

A disease or disorder is "alleviated" if the severity of a symptom of the disease or disorder, the frequency with which such a symptom is experienced by a patient, or both, is reduced.

As used herein, the term "composition" or "pharmaceutical composition" refers to a mixture of at least one compound useful within the invention with a pharmaceutically acceptable carrier. The pharmaceutical composition facilitates administration of the compound to a patient or subject. Multiple techniques of administering a compound exist in the art including, but not limited to, intravenous, subcutaneous, oral, aerosol, parenteral, ophthalmic, pulmonary and topical administration.

An "effective amount" or "therapeutically effective amount" of a compound is that amount of compound that is sufficient to provide a beneficial effect to the subject to which the compound is administered. An "effective amount" of a delivery vehicle is that amount sufficient to effectively bind or deliver a compound.

The terms "patient," "subject," "individual," and the like are used interchangeably herein, and refer to any animal, or cells thereof whether *in vitro* or *in situ,* amenable to the methods described herein. In certain non-limiting embodiments, the patient, subject or individual is a human.

As used herein, the term "pharmaceutically acceptable" refers to a material, such as a carrier or diluent, which does not abrogate the biological activity or properties of the compound, and is relatively non-toxic, i.e., the material may be administered to an individual without causing undesirable biological effects or interacting in a deleterious manner with any of the components of the composition in which it is contained.

As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the invention within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the invention, and not injurious to the patient. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound useful within the invention, and are physiologically acceptable to the patient. Supplementary active compounds may also be incorporated into the compositions. The "pharmaceutically acceptable carrier" may further include a pharmaceutically acceptable salt of the compound useful within the invention. Other additional ingredients that may be included in the pharmaceutical compositions used in the practice of the invention are known in the art and described, for example in Remington's Pharmaceutical Sciences (Genaro, Ed., Mack Publishing Co., 1985, Easton, PA), which is incorporated herein by reference.

Abbreviations used herein: RSH, thiol (e.g. N-acetyl-L-cysteine); RSNO, S - Nitrosothiol (e.g. S-nitroso-N-acetylcysteine), RS*, thiyl radical; RSSR, N-acetylcystinedisulfide; NO, nitric oxide; iNO, inhaled nitric oxide.

### General discussion

Pulmonary arterial hypertension (PAH) is a rare and highly debilitating disease. The morbidity and mortality rate (~ 7 years after diagnosis) is very high despite the use of costly ($75,000 to 250,000/year) pharmacologic drugs that interfere in NO, prostacyclin, and endothelin pathways. The use of traditional anti-hypertensive drugs through oral and IV routes cause several side effects including reducing systemic BP. Hence, drugs that can be administered directly into lungs in the form of gas or aerosolized are a better choice. In this context, inhaled NO (iNO) is a gold standard to provide a pulmonary specific vasodilator without compromising systemic blood pressure. Several clinical studies show that iNO clearly improves the ventilation-perfusion matching and lowers the pulmonary vascular resistance, thereby improving the oxygenation of blood in PAH and several PH-associated lung diseases. Presently, it has been used for the treatment of PPH/hypoxic lung failure in preterm and term infants and managing hypoxic lung failure and life-threatening PH for adults to avoid the need for more invasive and expensive ECMO treatment.

Current iNO therapy requires a complex and expensive (approximately $180/hour) system of gaseous NO storage cylinders, transportation, and devices to monitor and regulate the dilution and delivery of NO, O₂, and nitrogen dioxide (NO₂). Therefore, this therapy is available only in the intensive care units and operating rooms of established hospitals. It is possible that impurities in NO and the use of statistically underpowered subjects in clinical trials may be responsible for not observing clinical benefits of chronic treatment. Hence, there is a need for developing iNO therapy devices that are portable, less complex, and produce pure, cheap and desired levels of NO generation to avoid a diluting step where most of the NO₂ is generated and can be used immediately after preparation without storage. The present disclosure addresses this aforementioned need.

Since the FDA approved NO gas as a pharmaceutical agent, efforts are being made to develop portable NO-generating systems. However, in most systems, the NO generated in the reaction vessel is removed by streaming of N₂, air, or O₂ gas, which then must be purified to remove NO₂ that is generated as a result of NO reacting with oxygen. Hence additional steps for purification and monitoring the purity of the gases are required before administration to the patients. Monitoring devices need to be calibrated periodically to ensure the quality of iNO. In addition, patients need to buy NO cylinders from pharmacies as they do prescription medicine. All of these obstacles add to the cost of treatment. Hence, there is a need for development of better methods for generating NO as well as better systems for delivering NO.

Therefore, the technology of the present disclosure for NO generation is focused on simple methods that are inexpensive, and result in pure, controlled, and sustained levels of NO in the mixture of air or oxygen. The treatment can be available to a wider global population for use both in and out of hospital facilities. The systems and methods described herein can be used for such as ambulatory patients, in a patient's home, and in hospitals or clinics, including while patients are transporting within or between facilities.

In accordance with the purpose(s) of the present disclosure, as embodied and broadly described herein, embodiments of the present disclosure, in some aspects, relate to nitric oxide generation.

In general, embodiments of the present disclosure provide for methods of generating NO, systems and products for generating NO, systems for generating and delivering NO, and methods for delivering treatment including NO to a patient in need thereof.

### Methods

The present disclosure includes methods for chemically generating NO. Advantageously, the methods described herein generate pure NO gas, and may not require processing or purification to remove other reaction products. Unlike other methods and systems, the NO can be generated on demand at the point of delivery. The NO can be used for inhaled therapy or other medical uses.

In various embodiments, an inorganic nitrite (NO₂), specifically sodium nitrite (NaNO₂), is used as a precursor for NO. The NO₂ must be converted to NO. Nitrite protonates to nitrous acid (HNO₂) under acidic conditions. The pKa of this HNO₂ is 3.4. At pH below 3.0, the HNO₂ undergoes disproportionate reactions to form NO gas and nitrate. Thus, this procedure is not practical to synthesize NO for iNO therapy. In the pH region of 3 to 5, the HNO₂ can exist in the form of electrophilic nitrosoinum ion (NO⁺) (Eq. 1). In a particular embodiment, the reaction can occur in an acetate buffer having pH 3.5. Two methods, described below, have been developed to reduce this NO⁺ ion to NO.

NO₂⁻ + H⁺ ↔ HNO₂ + H⁺ ↔ NO⁺-H₂O (eq. 1)

NO⁺ ions rapidly reduce to NO in the presence of s electron donor compounds. In a first method for generating NO, ascorbic acid (RH) is used as an electron donor compound to reduce nitrosonium ion to NO (eq. 2). This NO can then be used as a source of iNO therapy.

NO⁺-H₂O + RH → NO + R' + H₂O (Eq. 2)

The method can include reacting a nitrite and an electron donor compound in an aqueous solution, wherein the reaction is performed under anaerobic conditions at a pH of about 3 to 3.5. In some embodiments, the nitrite is NaNO₂. The electron donor compound can be ascorbic acid. In some embodiments, the ascorbic acid concentration is about 0.25 M to 1 M. In some embodiments, the ratio of nitrite-to-ascorbic acid is about 1:3, about 1:4, or greater than 1:3.

Alternatively, a second method for NO generation is provided. NO⁺ ions readily complex with nucleophilic and weak electron donor compounds to form adducts. Low molecular weight thiols such as *N*-acetylcysteine can be used to react and form complexes with NO⁺ to generate S-nitrosoacetylcysteine (RSNO) (Eq. 3).

NO⁺-H₂O + RSH → RSNO + H₂O (Eq. 3)

This RSNO spontaneously decomposes to NO and corresponding disulfide (Eq. 4)

2RSNO → 2NO + 2 RSSR (Eq.4)

The disulfide remains in the reaction mixture and NO gas releases from the reaction mixture.

This method can include reacting a nitrite and a thiol in an aqueous solution. The reaction is performed under anaerobic conditions in the absence of light at a first pH of about 1 to 4.5. The reaction produces RSNO and at least a portion of the RSNO decomposes to NO.

In some embodiments, the thiol can be *N*-acetylcysteine, and the RSNO can be S-nitrosoacetylcysteine.

In some embodiments, the reaction can be performed in the presence of EDTA (a metal chelator).

RSNO is known to be unstable in the presence of reduced state metal ions such as cuprous Cu (I) ions. The RSNO is prepared and then rapidly degraded to NO and RS⁻ by treating with an amount of CuCl sufficient to degrade the RSNO to NO (Eq. 5).

RSNO + Cu(O) → RS⁻ + NO + Cu(II) (Eq. 5)

In some embodiments, the CuCl can be formed by reacting CuCl₂ and ascorbic acid.

In various embodiments, the thiol (also referred to as RSH) can be *e.g.* cysteine, *N-*acetylcysteine, glutathione, mercaptoethylamine, mercaptopropionic acid, *N-* acetyl-dl-pencillamine, Captopril, and the resulting RSNO can be *e.g.* S-nitrosocysteine, S-nitroso-N-acetylcysteine, S-nitrosoglutathione, S-nitrosomercaptoethylamine, s-nitrosopropionic acid, S-nitroso-N-acetylpenicillamine and S-nitrosocaptopril, respectively.

In various embodiments, the nitrite can be *e.g.* sodium nitrite, potassium nitrite calcium nitrite, zinc nitrite, copper nitrite, rubidium nitrite, strontium nitrite, barium nitrite, etc.

In some embodiments, the phenolic antioxidant can be *e.g.* quercetin, rutin, catechin, resveratrol, caffeic acid, curcumin, myricetin elagic acid, butylated hydroxytoluene, and eugenol. Antioxidant derivatives that include dextran, glucose or sulfonates show increased solubility without reduction in antioxidant activity.

In various embodiments, by manipulating the conditions under which the reaction is performed, the concentration of and rate at which NO is generated and/or delivered can be controlled, as can the duration of time over which the NO is generated and/or delivered. From a single reaction, the dose can be tuned from about 2ppm to about 40ppm of NO in air or O₂ over a period of about 1hour to 72 hours. In embodiments, the ratio of the nitrite to the thiol can be about 1:1 to about 2:1. The concentration can be tuned by adjusting the pH and ratios of the nitrite and the thiol to one another. For example, to sustain release of NO for about 1hr and wean off in about 6 hours, nitrite can be reacted with RSH at about 1:1 ratio in strong acidic conditions, where pH is < 1. To sustain release of the NO for about 6 hrs. and then wean it off in about 15 hrs., nitrite can be reacted with RSH at a ratio of about 1:1 in water. To sustain release of NO for about 10 hrs and wean off in about 25 hrs, nitrite can be reacted with NAC at the ratio of about 1.25: 1 in water. To sustain release of NO beyond 10 hrs, nitrite can be reacted with NAC at the ratio of about 2:1 in the presence of 50 mM quercetin in water.
To sustain release of NO for about 5 hrs, nitrite can be reacted with NAC at the ratio of about 1:1 in acetate buffer having a pH of about 2.0 followed by adjustment of the pH with NaOH to about 7.0. To sustain release of NO for about 7 hrs, nitrite can be reacted with NAC at the ratio of about 1.5:1 in acetate buffer having a pH of about 4.5.

In embodiments, the length of delivery of a dose, or period of time over which NO is generated at a substantially stable concentration, can be tuned by controlling the rate of release of NO after the reaction begins by raising the initial pH to a second pH. The second pH can be about 2 to 7, or about 4.5. For example, the initial pH can be about <1 and the secondary pH was also less than about 1 to obtain a sustained release of about 1 hr. For a sustained release of about 6 hrs, the initial pH can be about 2.0 and the secondary pH about 4.5. For a sustained release of about 10 hrs, the initial pH can be about 2.7 and secondary pH can be about 4.8. For a sustained release of about 15 hrs or more, the initial pH can be about 2.2 and the secondary pH can be about 5.2. For a sustained release of about 7 hrs, the initial pH can be about 4.5 and secondary pH can be about 4.5. For a sustained release of about 5 hrs, the initial pH can be about 2 and secondary pH can be about 7.0 adjusted with NaOH. A buffer, *e.g.* a potassium phosphate or NaOH can be included in the reaction to increase the second pH.

The reactants can be provided in various formulations, depending upon the desired rate of generation or duration of release of NO. In various embodiments, the reactants are provided in a combination of liquid and solid forms. For example, in one embodiment, solid form RSH and nitrite are provided in separate layers to a liquid media including HCl with EDTA. In another embodiment, the solids are provided in a tablet formation in which an outer layer includes NAC and an inner layer includes nitrite; the tablet is added to water with EDTA. In another embodiment, the solids are provided in a tablet formation in which an outer layer includes RSH and an inner layer includes nitrite; the tablet is added to and alkaline solution with EDTA. In another embodiment, the solids are provided in a tablet formation in which an outer layer includes RSH and an antioxidant and an inner layer includes nitrite; the tablet is added to water. In another embodiment, the solids are provided in a tablet formation in which a first layer includes NAC and a second layer includes nitrite; the tablet is added to an acetate buffer with EDTA. In another embodiment, the solids are provided in a solid formation in which a first layer includes NAC, a second layer includes nitrite, and a third layer includes NaOH; the solids are added to an acetate buffer with EDTA.

In yet another embodiment, the reactants can all be in a solid formulation, such as a tablet, that can be dispersed to release NO when provided to an aqueous solution. When nitrite is reacted with RSH, the initial rate of NO release is high in most of the reaction conditions. The baseline NO levels are stabilized in about 30 min. Once the baseline NO is stabilized, the water can be removed by flash evaporation. The residual contents of RSNO, unreacted nitrite and RSH, antioxidants and EDTA can be dried and stored under anaerobic conditions. Provided that the stability of this solid state is shelf stable for release of NO in sustained manner, it can be made into a tablet form to release varying concentrations of NO.

In some embodiments, all or part of the reactants can be provided in a solid formation, such as a tablet. For example, in one embodiment, solid form thiol and nitrite are provided in separate layers. In some embodiments, CuCl₂ and ascorbic acid can also be included in the tablet or provided as a second tablet. In other embodiments, ascorbic acid and nitrite can form the tablet.

### Systems

Embodiments of the present disclosure include an NO-generating system, wherein the system can receive reactants for generating NO as described as above. Advantageously, the system can be used to generate NO on demand without the need for large storage vessels or additional purification.

The NO-generating system can include a reaction vessel having a headspace. NO can be generated in the reaction vessel and the NO can be accumulated in the headspace. The reaction can be carried out in an aqueous solution in an airtight reaction vessel, where the reaction vessel is configured to provide anaerobic conditions for the reaction by applying a vacuum system to remove any gaseous particles in the reaction vessel headspace. The vessels can be composed of an acid- and high-pressure-resistant material (e.g. PTFE). The reaction vessel can have a volume capacity of about 20 mL to 100 mL, based on gas volume requirements of 50 mL to 1000 mL.

The system can include a gas evacuation port for the NO to exit the reaction vessel. Advantageously, the system allows for the vessel to be tipped at multiple angles without contaminating an evacuation line in the headspace with the reaction mixture nor exposing the delivery system, and thus the patient, to the reaction mixture. The NO can then be siphoned off via a narrow evacuation line that begins from the center of the vessel's headspace.

The vessel can, in some embodiments, have two compartments. The liquid portion of the reagent (e.g. water) can be placed in a bottom compartment, and a formulated chemical tablet can be placed in the upper compartment separated from each other. Thus, they will not react with each other. In some embodiments, turning an airtight O-ring-sealed cap allows the tablet to drop into the liquid portion, which then initiates a reaction to generate NO when it is required. The reaction vessel can have a side arm to siphon off NO gas into a reservoir.

The system can also include a stirring mechanism (*e.g*. a magnetic stir bar or bead and a stirrer) to facilitate even distribution of the reactants and increase the surface area to diffuse NO to the headspace.

The system can include NO storage for storing NO generated from the reaction vessel and dispensing it to mix with a carrier gas. The NO storage can be performed in the reaction vessel coupled to a gas regulator or using a separate NO reservoir. In some embodiments, the carrier gas can be oxygen or fresh air. Advantageously, the NO gas generated in the vessel can be delivered for about 10 hours to 72 hours. The time range dependent can be dependent on the amount of reactant, the dilution in the carrier gas, or both. Unlike other systems, rapid and pure NO can be generated and stored locally in small volumes on demand.

In embodiments wherein the NO storage is the reaction vessel coupled to a gas regulator, the pressure generated by the NO generation reaction in the reaction vessel can be used as a driving force to deliver the NO. For example, the NO can be generated by using an appropriate amount of reagents in the fixed space of the vessel and can reach a pressure of about to 50 psi to 100 psi. A miniature two-stage in-line gas regulator can be used to reduce and maintain a constant outlet pressure of 0 to 15 psi. The desired amount of NO can be mixed with carrier gas using an electronically/manually control valve for a sustained period of time until the stock NO pressure decreases to 15 psi. Approximately 85% of the stock NO can be delivered using the gas regulator.

In embodiments wherein the NO storage is a NO reservoir, a gastight syringe can be used as a NO reservoir that dispenses NO to mix with carrier gas. The required amount of NO is generated in the limited space of the reaction vessel with an appropriate amount of reagents. The NO is transferred into the syringe barrel when a plunger is pushed by the mechanical force of pressure. Therapeutic doses of NO from the syringe are dispensed by an electronically controlled motor pump. The stock NO that is over the reaction vessel's headspace can be delivered using the NO reservoir. Advantageously, this type of syringe reservoir allows for delivery of 100% NO gas mixed with a carrier gas to a patient.

The system can include a mixing chamber. The chamber provides for instantaneous mixing whereby NO is sprayed into flowing oxygen gas in the mixing chamber in order to reduce dilution time. The system allows for mixing of 100% NO with oxygen gas at a safe level of NO₂ formation.

Before inhalation by patients, NO gas must be diluted with carrier gas into small therapeutic doses (1 to 80 ppm). Existing inhalation therapy systems rely on pre-diluted NO stock compressed into large cylinders. This is because NO reacts rapidly with O₂ to generate toxic NO₂ gas. The rate of this reaction equals the square of the NO concentration and is linear to the oxygen concentration. The calculated rate constant for the reaction of NO with oxygen is 7.3 × 10³ mol/L⁻² s⁻¹ or 1.19 ×10⁻¹¹ ppm⁻² s⁻¹. Based on these rate kinetics, and supported by several laboratory experiments, 80 ppm NO in 85% oxygen generates 0.14 ppm/s NO₂. However, if dilution of 800 ppm NO with O₂ to 80 ppm generates about 1.4 ppm, that is more than 10 times higher than the calculated value because the initial concentration of NO and subsequent reactions with O₂ are higher before the dilution is completed. The cylinders used in existing inhalation therapy systems is diluted 1250 times with nitrogen to 800 ppm. In spite of this dilution, unsafe limits of NO₂ generation have been reported, particularly when diluted to the more than 20 ppm that is often required. If undiluted 100% stock NO is diluted to 80 ppm, it is expected to generate 1.4 ppm NO₂ 1250² times (~1.56 million) faster than 800 ppm, equivalent to about 1 microsecond. Therefore the 100% NO needs to be diluted to 80 ppm in less than 1 microsecond in order to avoid the NO reaction with O₂ at the mixing point. The mixing chamber and system described herein can achieve the needed dilution speed.

Embodiments of the present disclosure also include NO generating systems that can include a reaction vessel having a headspace for NO accumulation, a NO storage system, and a gas mixing chamber. NO gas generated in the reaction vessel can be transferred to the NO storage system via pressure generated by harvested NO gas. The desired concentrations of NO can be dispensed into the mixing chamber to dilute with carrier gas using an infusion pump.

In some embodiments, the reaction vessel is the NO storage system. In other words, the reaction vessel can also serve as the NO storage system without the need for a separate storage system. NO can be both generated and stored in a single vessel. The reaction vessel can withstand gas pressure higher than 1 atm, and the pressurized NO gas can be delivered from the reaction vessel to the mixing chamber at a desired flow rate via a gas pressure gauge regulator.

In some embodiments, the gas regulator can have an inlet and an outlet, and the gas regulator can maintain a constant outlet pressure of 0 to 15 psi.

Embodiments of the present disclosure also include a reactant module for NO generation. Such a module can be in the form of a tablet, cartridge, or other formation containing the reactants for introduction to the reaction vessel. The module can include a nitrite, RSH, a phenolic antioxidant, EDTA, and aqueous solution as described above so that the nitrite, RSH and antioxidant are not in direct physical contact with the aqueous solution. The module can be configured such that upon activation of the module, the reactants combine and react to produce NO. For example, in certain embodiments the reactants can be in a powdered form with binders or buffers separating the nitrite from the thiol in a tablet form as described above. Upon introduction of the tablet into an aqueous solution in the reaction vessel described herein, the tablet would dissolve and the reaction initiated. A buffer, as described above, can also be included. In another embodiment, a cartridge with sections to keep liquid and solid reactants separated before introduction to the reaction vessel can be used.

Embodiments of the present disclosure provide systems for NO delivery, such as to a patient. The NO delivery system can include a breathing tube, electrochemical gas detectors (ECD), electrical, and electronic systems. The NO delivery system can deliver the diluted NO and oxygen mix from the mixing chamber described above. The communication system can have a user input device that enables the programing of desired parameters, a central processing unit to perform computational functions, and stored memory for machine-executable instructions. The ECD can be used to determine NO and NO₂ formed as a result of NO reaction with O₂ in the mixing chamber. The user input device can communicate with the CPU, and the CPU can communicate with stored memory. The CPU can communicate electronically with the ECD, the syringe reservoir's motor pump or gas pressure regulator to dispense NO gas, "on /off" valves, and the vacuum pump to make the whole system anaerobic just before use.

In some embodiments, the NO delivery system can be combined with the NO generating systems as described above to form a system for both NO generation and delivery. Advantageously, the system is smaller than traditional systems. For example, the system can be small enough to be portable, for use on a benchtop, or outside of a hospital setting (e.g. home or office use). In one embodiment, for example, the NO can be delivered to the patient as an inhalant via a patient delivery tube (such as a mask or nasal plugs). The patient delivery tube can be connected to a pump (e.g. a vacuum pump) configured to extract NO from the vessel headspace.

In various embodiments, the NO generating and delivery system further includes a gas flow regulator to regulate the amount of NO gas reaching the patient. In various embodiments, the delivery system includes one or more elements, such as a NO₂ filter coupled to the patient-delivery NO tube, an oxygen cylinder configured to mix oxygen with the extracted NO for delivery to the patient, and a monitor/detector able to detect such as NO, O₂, and NO₂. In certain embodiments, higher concentrations of NO may be generated and then diluted to desired levels using an injector module. The dose levels can be increased or decreased during the treatment.

Embodiments of the present disclosure include methods of treating a patient in need of inhaled NO. The inhaled NO can be generated as described above. The NO can be generated in a reaction vessel, mixed with a carrier gas, and provided via a tube to the patient for inhalation. The patient can have at least one of a pulmonary arterial disease, a lung disease or condition (e.g. Acute Respiratory Distress Syndrome (ARDS), Chronic Obstructive Pulmonary Disease (COPD)), sleep apnea, high altitude hypoxemia an infection, can be in need of cardiac surgery, or recovering from a cardiac surgery. The extracted NO can be monitored for contaminants via a detector before the NO is provided to the patient.

### EXAMPLES

Now having described the embodiments of the disclosure, in general, the examples describe some additional embodiments. While embodiments of the present disclosure are described in connection with the example and the corresponding text and figures, there is no intent to limit embodiments of the disclosure to these descriptions. On the contrary, the intent is to cover all alternatives, modifications, and equivalents included within the spirit and scope of embodiments of the present disclosure.

### Example 1: Systems for NO generation and delivery

Nitrite (NO₂⁻) has been used as a precursor for NO, but no existing technology has reached the clinical stage, due to the difficulty in converting nitrite to NO from sustained periods of time, and because of dangerous levels of generated NO₂. The methods and systems described herein also use nitrite as a precursor to synthesize NO gas but use different methods and systems. Nitrite is known to protonate to nitrous acid (HNO₂) in weak acidic conditions. The pKa of HNO₂ is 3.4. In this pH region, HNO₂ also exists in the intermediate species of nitrosonium ion (HNO₂ ↔ NO⁺-H₂O), which can be reduced rapidly to NO by any electron donor compound such as ascorbic acid, polyphenols, etc. We used ascorbic acid as a reducing agent to generate bulk amounts of NO that could be used as inhaled NO. NO⁺ also readily complexes with thiol residue molecules to form S-nitrosothiols. A low-molecular-weight thiol compound, N-acetylcysteine, is used to react with this NO⁺ to form S-nitrosoacetylcysteine (RSNO). This RSNO decomposes spontaneously to NO as a sustained source of NO or rapidly degrades by cuprous chloride (CuCl) to create a bulk source of NO.

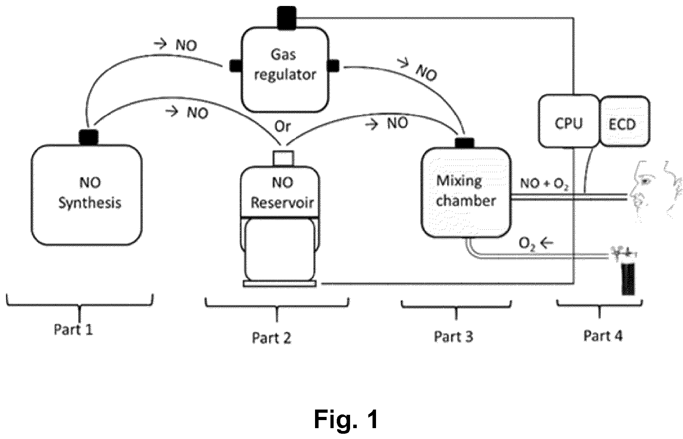

An overview of NO generation and delivery according to the present disclosure is provided in Figure 1. For ease of reference, the method and systems can be divided into four general parts. Part 1 is for synthesis of NO. Part 2 is for storing NO and dispensing it or using a gas pressure regulator to restrict the gas flow to therapeutic levels. Part 3 is for mixing NO with oxygen, and part 4 is a patient delivery system including a gas-monitoring device, central processing unit, and breathing tubes.

Part 1 of the device, for NO synthesis, can include a disposable unit. The disposable unit can be such as a custom-made 50 ml glass reaction vessels or made using 3D printing technology.

Part 2 of the device can include a syringe base NO reservoir. Airtight Hamilton gas syringes (25 ml or 100ml) have been used as NO reservoir for experiments in laboratory. An infusion syringe pump (Harvard Apparatus type 2000) is used to dispense the NO from the reservoir. A miniature gas regulator has been used for gas flow control.

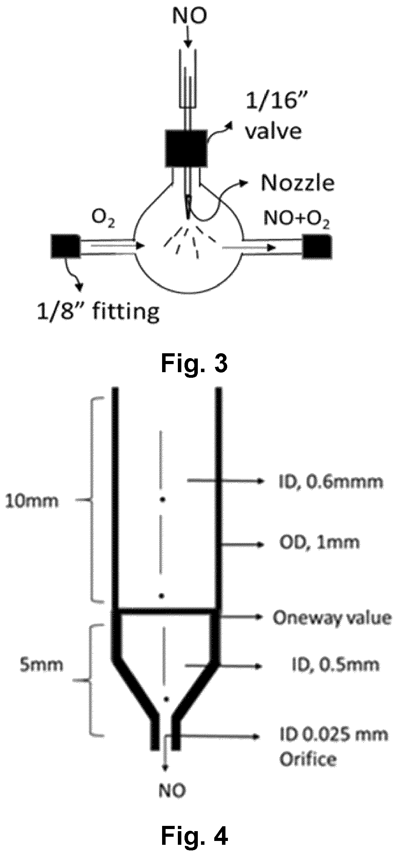

Part 3 of the device can include a custom built 10 ml volume glass vessel similar to that shown in Figure 3. The nozzle can be varying sizes of (100 to 300 micrometer) tubing or can be such as a hypodermic needle with an inserting tip. The inserting tip can have a hole size of about 10 to about 50 µm or about 25 µm.

### Example 2: NO generating device

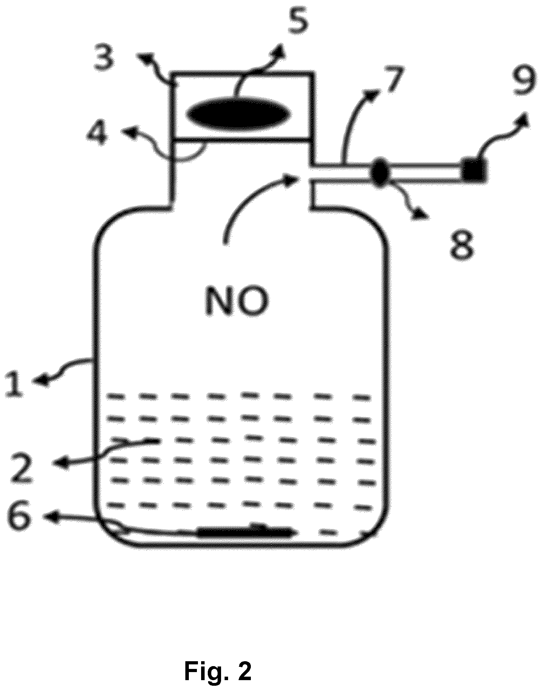

*Disposable Unit:* Figure 2 illustrates an embodiment of a reaction vessel, also called a disposable unit, in which NO can be generated under strict anaerobic conditions. Eliminating oxygen avoids the possibility of NO₂ formation. It is made up of an acid-resistant PTFE material and has a volume capacity of 50 to 100 ml based on the amount of NO gas generation required (50 ml to 1000 ml). It consists of two compartments. The first one is a major compartment (1) that holds the liquid portion (2) of the reagent. A formulated chemical capsule (5) is placed into the second compartment (3). Thus, the liquid and capsule cannot interact. When the airtight O-ring-sealed cap (4) is turned, the capsule is released into the liquid portion to initiate a reaction to generate NO. The reaction vessel has a gas exit line (7) that ends at an airtight push-to-control fitting with an internal seal valve or O-ring (9). A hydrophobic filter (8) is placed in the gas exit line (7) to prevent any moisture from entering into the gas reservoir. The solution is stirred with a magnetic bar (6) to facilitate the dissolution of chemical and initiate the reaction to generate NO. The NO gas pressure in the vessel increases as a result of NO gas accumulation in the limited space.

*Mixing Chamber:* Figure 3 illustrates one possible embodiment of the mixing chamber. A round glass vessel with a capacity of 2.5 mL to 10 mL is used for mixing the NO in carrier gas. It has two side-arms with 1/8 inch Swagelok^{®} fittings for PTFE tubing having 1/8" outer diameter x 1/16" inner diameter. One arm is used as an inlet for carrier gas and the other arm is used as an outlet for carrier gas mixed with NO. The top port is connected with a nozzle to introduce the sample. Nozzles are used to increase the flow velocity of fluids (liquids or air) while exiting, from its small diameter tip with the expense of its pressure energy. A cone-shaped nozzle is designed to spray the NO into the mixing chamber as shown in Figure 4. This nozzle is connected to a NO gas line that originates from the syringe reservoir using a gastight fitting (Swagelok^{®}). The nozzle tip is inserted to in such a way to reach middle of the mixing chamber. The mixing chamber's gas inlet is connected to a carrier gas cylinder, and the outlet is connected to a gas inspiratory tube. The force is applied on the syringe plunger to increase the pressure on the NO to spray it into the mixing chamber where it mixes with flowing carrier gas. The flow rate of the carrier gas varies from 1 LPM to 6 LPM. The velocity of the carrier gas is calculated based on the flow rate and diameter of the tube. For example, if the flow rate of carrier gas is 2, 4, and 6 LPM, the velocity would be 16.8, 33, and 50 m/sec or 16.8, 33, and 50 µm/µsec, respectively. Similarly, the velocity of the NO gas flow rate is calculated using the pressure difference between primary at the head and secondary at the tip and diameter of the orifice. The mixing of NO and oxygen at such high velocities reduces the dilution time with concomitant reduction in NO₂ generation.

Figure 4 illustrates an embodiment of the nozzle that is used to increase the flow velocity of NO while exiting from its small diameter orifice with the expense of its pressure energy. A cone-shaped nozzle is designed to spray the NO into the mixing chamber. Seamless steel metal that is resistant to NO oxidation is used to make the nozzle. The nozzle has 0.6 mm inner diameter, 1 mm outer diameter, and 0.2 mm thickness. The nozzle's orifice inner diameter is about 0.025 mm, and the length is about 15 mm. Without being bound by theory, Applicant believes that nozzle orifices having an inner diameter of 100 nm or less (*e.g.,* between about 10 nm and about 100 nm) would produce sufficient NO velocity to avoid unacceptable NO₂ production. The nozzle has a one-way valve toward the chamber side between head and cone tip. The nozzle can increase the flow velocity of fluids. In order to decrease the NO reaction with O₂, a small volume of NO (*e.g.* in µL amounts) is mixed instantaneously in a large volume of O₂ (*e.g.* in liter amounts). The measurements described herein and shown in the figure are examples and can be modified as can be envisioned by one of ordinary skill in the art.

*NO storage system:* The NO storage system can be either a syringe reservoir or a gas regulating/dispensing unit. The syringe reservoir can be a gastight syringe with a volume capacity of 10, 25, 50, or 100 ml, depending on the amount of NO gas required for storage and delivery. The gas regulating/dispensing unit can be a programmable infusion pump used to dispense the NO gas from the syringe reservoir.

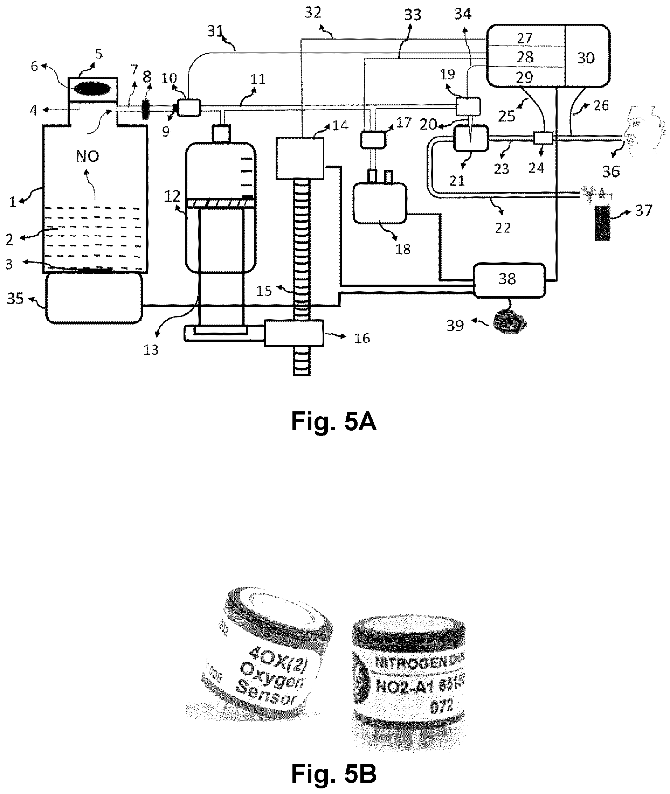

*Base unit using reservoir as NO dispenser.* One embodiment of the system is shown in Figure 5A. The system includes a reaction vessel (1) to contain a liquid portion (2). A screw cap (4) can seal the vessel (1), and a chemical capsule (6) can be contained in a capsule compartment (5) for addition to the liquid portion (2). A gas exit line (7) leads to a hydrophobic filter (8) connecting to gas exit line (9) and inserted into the inlet "on/off" valve (10). The outlet of valve (10) is connected to a gas line of 1/16-inch PTFE tubing (11). The end of this tube is connected to the "on/off" valve (19). The syringe reservoir (12) front nose and "on/off" valve (17) inlet are connected to gas line tubing via T-joints. The motor gear unit (14) leads to a threaded rotational spindle (15) to which a movable drive shaft (16) is attached; this portion of the system controls the dose and flow levels. The rear-end of the syringe plunger (13) is inserted into the holder that in turn inserts into a movable drive shaft (16). The vacuum pump (18) inlet is connected to the outlet of the "on/off" valve (17). The nozzle's head (20) is inserted into the "on/off" valve (19) exit gas line tubing. This nozzle is positioned into the mixing chamber (21) by gastight 1/8-inch NPT fittings (Swagelok^{®}). The NO carrier gas (O₂ or air) line (37) is connected to the mixing chamber's inlet (21), and the mixing chamber's outlet is connected to a breathing tube (23). A flow sensor, (24) is placed in the inspiratory gas line to measure the flow rate. A sampling gas line (26) positioned just before the mask (36) withdraws gas from the breathing tube for measurement of NO and NO₂ by an electrochemical detector (ECD). The programmable computer that controls the operating system (memory 27, CPU 28, input device 29) is located adjacent to the gas sensor device (30). This computer consists of a user input (e.g. keyboard device) (29) for entering the patient's dosing information, memory (27) to store the program instructions, and a central processing unit (28) that processes the instructions and communicates with the device. The CPU communicates back and forth to the ECD. The CPU further communicates electronically with the "on/off" gas line valves (10, 17,19), motor gear (14), and a cable (25) connecting a gas flow rate sensor and the CPU as per the programmed instructions. Communication cable (32) communicates between CPU (28) and the infusion pump motor (14, 15, 16); communication cable (32) controls the dispensing amount of the NO into the O₂. Cable (33) controls the on/off switch of vacuum pump (18) and cable (34) controls the on/off switch of the mixing chamber (21). The magnetic stirrer or vibrator (3) is placed in the device in such a way that the bottom portion of the disposable reaction vessel (1) is positioned on it. A battery backup (39) and power port (38) provide regular AC power to the electronic control system (28), gas analyzer (30), motor pumps, (14, 18), and magnetic stirrer (35) controls magnetic stir bar (3). Figure 5B shows an example of a nitric oxide and nitrogen dioxide dual gas electrochemical sensor device (30) that can be used in the system.

*Function of the device:* The disposable NO-generating unit gas outlet (9) is inserted into the base unit "on/off" valve's inlet (10) to connect airtight. The operating program is installed in the computer memory. The power to the instrument is turned on. The stir bar in the reaction vessel initiates stirring the solution. The user sets the desired NO value levels and patient information on the input device. The input device communicates with programmed memory to follow the instructions and then communicates with the CPU to perform the series of instructions for device functions. The "on/off" valves (10, 17) and vacuum pump (18) turn to the "on" position from the off position. The vacuum pump (18) removes the air from the reaction vessel (1) and from the gas line (11) for 5 minutes. After 5 minutes, the valve (17) turns to the "off" position, and the vacuum pump (18) also turns off. The chemical capsule (6) in the reaction vessel is released to mix with the liquid portion and generate a desired amount of NO gas. Any NO₂ generated in the reaction vessel by reaction of NO with traces of O₂ diffuses back into the liquid portion and converts back to NO. Then the valve (10) is opened to transfer NO from the reaction vessel to the syringe barrel until the plunger reaches the stopping point (16). The pressure in the syringe is about 10% higher than atmospheric presser (14.7 psi) because of some resistance in the syringe plunger. The oxygen supply connection (37) to the mixing chamber is turned on, and the user sets the desired gas flow rate between about 1 L/min to 6 L/min. The gas sensor (25) measures the exact gas flow rate and communicates this information to the CPU (28) to calculate the NO dose to be delivered. The valve (19) is turned to the "on" position, allowing the NO nozzle to inject into the mixing chamber to dilute with carrier gas, oxygen. The CPU is in communication with the syringe motor pump (14) to push the plunger (13), which increases the pressure in the barrel to dispense NO into the mixing chamber. The ECD withdraws the sampling gas (26), determines the NO and NO₂, and displays these values on the screen. The CPU communicates with the gas sensors and then with the reservoir motor (14) to regulate the delivery of NO to the set values.

In this example, the range of NO doses can be about 1ppm to 80 ppm with an average dose of about 20 ppm. The supplemental oxygen flow range is about 1 to 6 Liters /min.

### Example 3: NO Delivery Systems

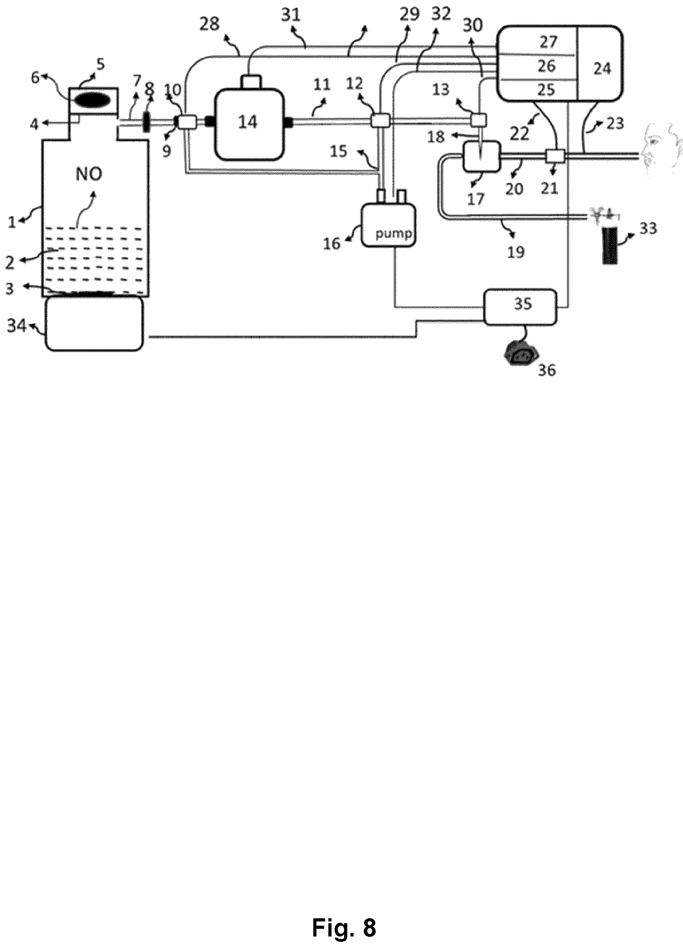

*Using gas regulator to dispense NO:* In the example shown in Figure 8, after initiating the instrument, the vacuum pump 16 turns on and simultaneously "on/off" valves 10 and 12 turns to on position for 3 min to evacuate the air in the gas tubing, regulator and the reaction vessel and then turns to "off" position. In the off position, the gas line makes a connection from valve 10 to 13. The desired amount of NO is generated in the vessel using appropriate amount of reagents to reach 150 psi for 5 min. The regulator 14 reduces this pressure and maintain the constant outlet pressure to 0 to 15 psi. After 5 min the on/off valve 13 changes to "on" position towards the mixing chamber. The outlet gas flow rate to mix with carrier gas is controlled by 7 increase or decrease the pressure control using electronically controlled regulator knob. The rest of the procedures similar to Figure 5A.

*NO delivery using regulation of gas directly from the reaction vessel:* In another embodiment of the device shown in Figure 8, compressed gas pressure is used as a mechanical force to deliver the NO directly from the reaction vessel without transferring to a reservoir. An inline two-stage gas regulator (Swagelok^{®} Company) that can be controlled electronically is used to reduce and maintain the constant outlet pressure between 0 and 15 psi while delivering NO gas. The gas regulator (14) is placed in the gas line (11) between "on/off" valves 10 and 12. The "on/off" valves (10 and 12) are connected to the vacuum pump inlet (16) using a T-joint (15) to remove air from the gas line and the system. The gas regulators, vacuum pump, and "on/off" values communicate electronically with the CPU similar to the syringe reservior delivery.

Operating the device: The operating program is installed in a memory disk. The power to the instrument is turned on, and the stir bar in the reaction vessel begins stirring the solution. The user sets the desired levels of NO and patient information on the input device. The vacuum pump will turn on for 10 minutes to remove air from the reaction vessel and gas lines, including the gas present in the internal space of the regulators. Then the reaction initiates to generate NO gas up to the maximum pressure of 100 psi, in which 80% of NO can be delivered to patients. The outlet pressure is kept between 0 and 15 psi, and the flow regulatory needle valve is used to deliver the desired NO volume to mix with carrier gas in the mixing chamber. The gas regulator enables delivery of 10 ppm to 200 ppm NO for sustained periods of time until the inlet pressure declines to 20 psi.

Table 1 shows the NO₂ determination in NO carrier gas. NO₂ is measured by an electrochemical detector. These NO₂ levels using the gas regulator are a bit higher than those formed when the syringe reservoir is used to deliver the NO. The NO₂ is approximately 0.56 ppm in nitrogen carrier gas, indicating that the system has some air leak. When air is used as the carrier, NO₂ levels are 0.85 ppm at 20 ppm NO and 1.7 ppm at 40 ppm NO. When O₂ is used as the carrier, NO₂ levels are 1.76 ppm at 20 ppm NO and 3.2 ppm at 40 ppm NO. These values are a bit higher than the safety limits of <1 ppm. However, it is possible to place an NO₂ scrubber in the breathing gas line to reduce the NO₂ to safe limits.

**Table 1. NO₂ determination in NO carrier gas**

| Gas type | Gas flow rate | NO (20 ppm) | NO (40ppm) |
|---|---|---|---|
| Nitrogen | 2L/min | 0.56±0.03 | 1.1± 0.05 |
| Air | 2L/min | 0.85 ± 0.036 | 1.7± 0.087 |
| Nitrogen | 2L/min | 1.76± 0.074 | 3.2± 0.1 |

### Example 4: Portable device for delivery of NO

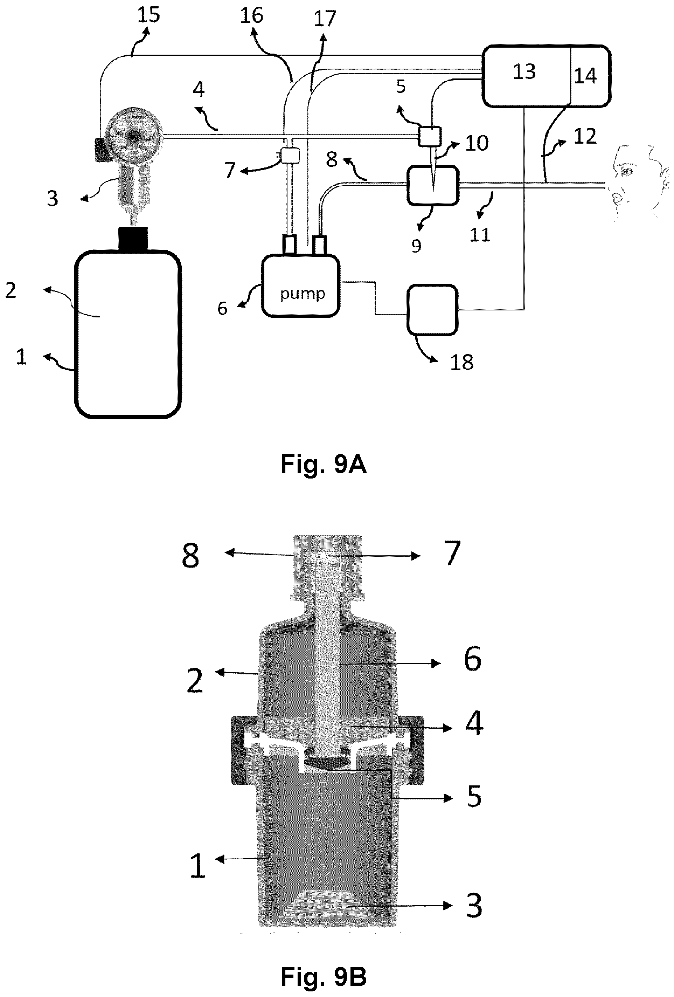

A schematic of a portable device is shown in Figure 9A. This benchtop device consists of two units. One is a NO-generating unit and the second is a NO-delivery unit. The NO-generating unit consists of two separate chambers: one which holds the chemicals and one which holds the liquids. When NO is needed, a mechanism is initiated that mixes the chemical into the liquid to trigger the NO-producing reaction. This unit is mounted to a gas exit line that is aligned with the NO-delivery system.

The NO-delivery unit consists of a NO gas reservoir, NO-dispensing motor system, gas evacuation system, mixing chamber with nozzle system, and gas sensor system. A central processing unit regulates the electronic control system and magnetic stirrer. The instrument operates via a programmable electronically controlled system.

The benchtop system can be configured as a portable system by miniaturizing the components of the benchtop device with two main alterations. A miniature air pump is used as a source of ambient air to carry the NO gas and the portable device is designed to separate the delivery system from the NO-generating unit and the magnetic stirrer.

About 200 ml of NO is compressed to 20 ml and stored in a minicylinder that is equivalent to 10M ATM pressure. The custom-made dual stage miniature gas regulator (70g) with adjustable orifice can be used to regulate gas flow rate (Beswick Engineering Co., Inc. and Parker Hannifin Corporation Precision Fluidics Division). The device can include a custom disposable gas cylinder with 20 ml volume capacity (1) and matching inlet miniature gas regulator (3). NO gas (2) is compressed into the can at the pressure of about 150 to 200 psi. The gas regulator's outlet is connected to NO gas line tubing (4) and its end connected to "on/off" valve (5). Mini-vacuum pump (6) gas inlet can be connected with PTFE tubing to the gas line tubing (4) using such as a T joint. "On/off" valve (7) is placed near this T joint. When this valve is in the "on" position, it makes a connection between gas line (4) to the inlet of pump (6) to remove oxygen. When the valve is in the "off" position, it opens to outside for the source of atmospheric air to pump outlet. The outlet of pump (6) is connected to mixing chamber's (9) inlet through tubing (8) for air as NO carrier gas. The nozzle (10) is inserted into "on/off" valve (5) outlet gas tubing. This nozzle is placed in the mixing chamber (9) using airtight fittings (Swagelok^{®}). The mixing chamber's exit is connected to breathing tube (11). Programmable control unit (13) and electrochemical detector (14) are included. The sampling gas line (12) is connected to ECD (14) to draw the NO gas to measure the NO and NO₂. The programmable control unit communicates with "on/off" valves (5, 7), cables (15,16) and air pump on/off switch (17) to execute the device functions. Cable (15) communicates with the gas regulator knob to control pressure, and cable (16) controls the on/off switch (7) for the vacuum pump (6). The device can include a power source (18), such as a rechargeable battery.

In a particular embodiment, the single-use reaction vessel holds and combines the reagents responsible for generating the on-demand NO gas. The design uses a custom, dual-reservoir container (65ml volume) where the lower chemical chamber and upper liquid chamber are separated by a port. The port is plugged with a stemmed plunger which extends to the top of the vessel and capped with a standard septum. The reaction vessel can be loaded into the benchtop prototype in a custom-designed, actuated, receptacle. At the start of therapy simulation, a motor-driven needle will pierce the septum and drive the internal plunger through the port, connecting the two chambers. Once connected, the two-part reaction will start to generate NO gas and pressurize the reaction vessel. A magnetic stirrer located in the bottom of the receptacle assists in catalyzing the reaction.

Another embodiment of the mixing chamber for the benchtop device is shown in Figure 9B. This example has a reaction vessel with a lower chamber (1), an upper chamber (2), chemicals (3), liquid (4), plug (5), plunger (6), rubber cap (7), and cap (8).

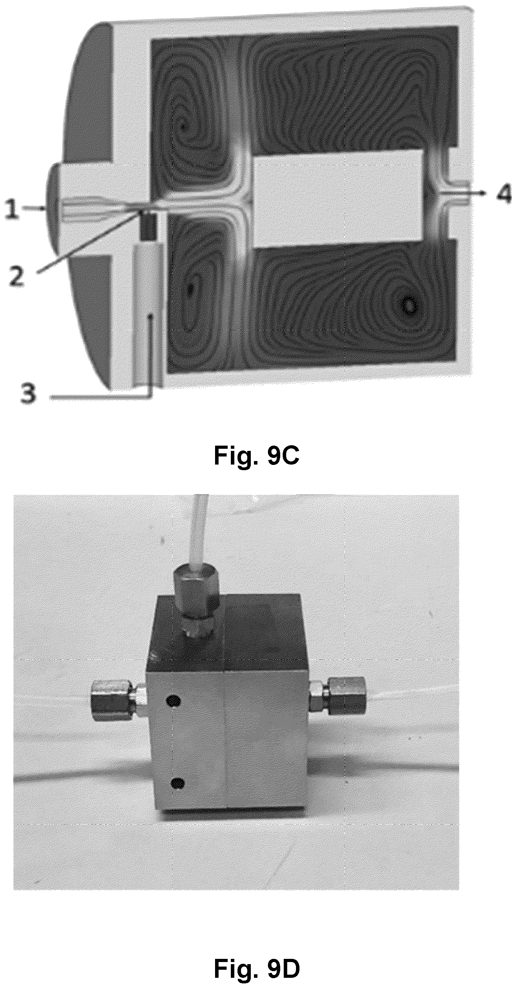

Figure 9C provides a schematic of a mixing chamber and Figure 9D is an example of a mixing chamber. In the mixing chamber, the highly concentrated NO gas is injected into the external gas flow (O₂ or air). Improper mixing can generate toxic levels of NO₂ and must be minimized by rapidly diluting the NO into the external gas. Due to the extremely low volume ratios of the two gasses (0-80 parts per million), the mixing must be precisely controlled.

It has been demonstrated that most of the NO₂ is generated at the mixing point before the dilution is complete. To improve upon this, two mixing chambers were designed and tested in this phase. The first was a replication of the laboratory glass-chamber using a stainless-steel housing and commercially available sapphire orifice. Computational Fluid Dynamics was used to simulate the system to better understand the mechanism by which the NO was mixing into the O₂. Simulations showed that the rapid volume expansion of the O₂ flow into the large mixing chamber volume developed a turbulent flow. A dynamic eddy likely develops around the region of the NO orifice and acts to disperse the NO flow in a random spiral pattern, preventing the accumulation of concentrated NO forming.

To make a more controlled mixing environment, a second mixing chamber was designed to increase the velocity of the NO (3) and external gas (1). The NO velocity increase is accomplished in the same manner via a 25-micron orifice (2), while the external gas velocity increase is accomplished with a necked-down channel. The exit points of the orifice and channel are placed in close proximity in order to maximize the mixing velocities, while also entering into a low-pressure region of the flow to prevent back-pressure. Immediately after the mixing point, the combined gases enter a larger chamber where the NO gas can be fully diluted into the stream before exiting the mixing chamber as Oxygen and NO (4).

Experiments confirmed that the second chamber design produced more reliable results, with lower NO₂ generation, over a wider range of flows.

In order for the mixing to be successful, the pressure of the NO gas must be higher than the pressure of the mixing chamber. Failure to do so will result in back flow through the orifice and significant levels of NO₂ generation. Therefore, the dose range the system can provide is bounded by the minimum required pressure of the NO (relative to the mixing chamber), and the maximum reasonable pressure the system can generate on the NO using the infusion pump. The range can be altered to some extent by changing the orifice diameter but will always be bound.

In an example having a 25-micron orifice, the velocity can be about 0.1 m/s to 50 m/s, or about 0.1 m/s to 30 m/s. For example, for 2 Lpm of O₂ and NO of 5 ppm, the velocity can be about 0.3 m/s. For 10 Lpm of O₂ and NO of 80 ppm, the velocity can be about 27.1 m/s.

### Example 5: NO delivery system

As described above, in order to deliver NO effectively to a patient, a nitric oxide-generating and -delivery system can be used. In various embodiments, the NO can be generated via the nitric oxide generating system described herein and stored for later use in patient delivery or generated for use in non-patient delivery systems. In other embodiments, the NO generating system is combined with a delivery system, in which the generated NO can be generated and delivered to a patient in tandem.

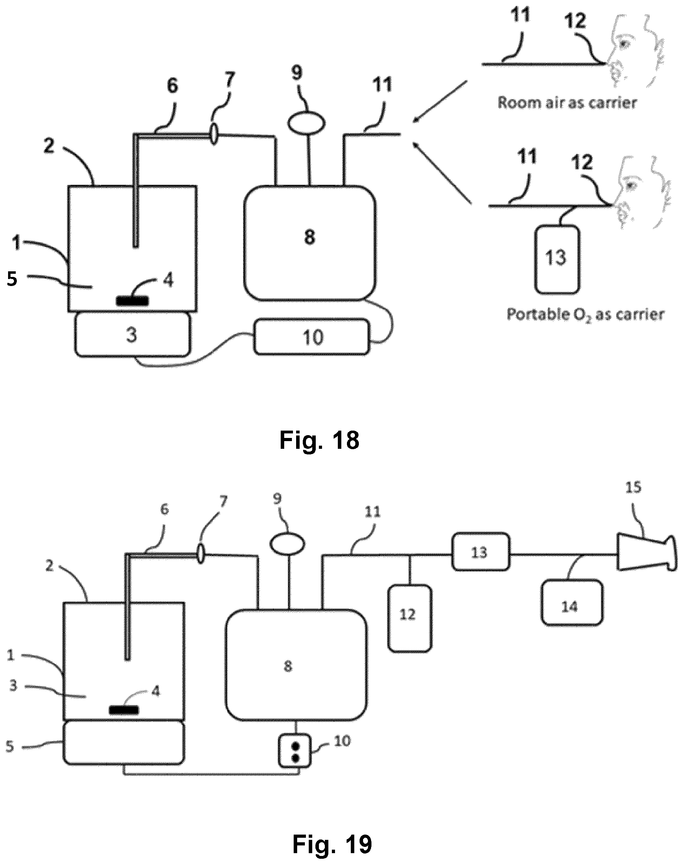

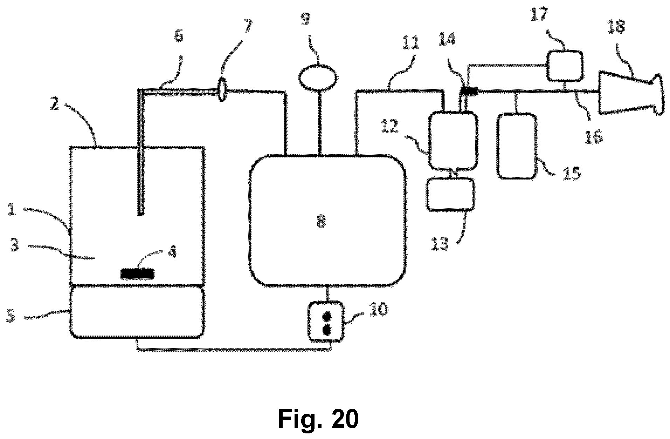

Figures 18, 19, and 20 provide schematics of some possible embodiments of NO delivery systems. Figure 18 shows an embodiment of a portable delivery system which may be used by ambulatory patients, which could enable treatment in home or other non-hospital settings. Figure 19 is another embodiment of a portable benchtop NO generating system, which could be used in scenarios including emergency conditions at homes, ambulatory, clinics, community hospitals and the like. Such a system, while potentially larger than the personal portable system of Figure 18, would be small and inexpensive enough for use in non-hospital clinical settings and could generate enough NO to treat multiple patients per day. A device such as shown in Figure 20 can be used to delivery varying doses of NO and increase or decrease the NO doses during the treatment using injector module that is controlled by NO monitoring device.

The systems consist of a base unit and a secondary unit, where the secondary unit can be disposable. The base unit for ambulatory patients can be composed of a battery-powered micro-diaphragm vacuum pump, mini-magnetic stirrer, and nasal cannula. The disposable unit consists of a reaction vessel mounted with a vacuum line, sample inlet screw cap port, and chemicals to generate NO. This vacuum line will be aligned to the vacuum pump inlet to remove NO gas. The base unit for the tabletop unit will be composed of an AC-powered vacuum pump, magnetic stirrer, and nasal cannula with optional of oxygen cylinder, NO scrubber and NO, NO₂ and O₂ detector.

The secondary units can be the same for the two devices. Figure 18 provides a possible embodiment of a nitric oxide generation system for portable device including a reaction vessel 1, a receptacle 2, magnetic stirrer 3, magnetic bead or stirrer bar 4, NO generating reaction mixture (reagents) 5, a vacuum pump 8 to pull the NO through the vacuum line 6, a gas flow regulator (gauge) 9, NO in the air exit tube (Nasal tube) 11, optional oxygen cylinder 13. Also included can be a filter 7, a chargeable battery 10, a nasal cannula 11, and nasal plugs 12.

The benchtop device (Figures 18 and 19) can have the same disposable unit (elements 1 to 7), an AC powered mini-vacuum pump, optional O₂ gas 12, NO₂ scrubber cartridge 13, NO, NO₂ O₂ detector 14, nasal plugs or face mask, 15. Another benchtop device design to deliver varying doses of NO (Figure 20) can contain an additional NO gas reservoir and trap 12 &13, and an injector module 14 to deliver desired amounts. The reaction vessel is configured to provide anaerobic conditions under which the reaction is performed. In the reaction vessel 1, a nitrite, and a thiol in aqueous solution can be reacted and stirred with the magnetic stirrer 3 and magnetic bead or bar 4 to generate the NO generating mixture 3. The resultant NO can be drawn through an evacuation line by a vacuum pump 8. The flow of the NO through the air exit tube 11 can be regulated by a gas flow regulator 9 before reaching a patient via a mask or nasal plugs 15. The NO flow can be configured to mix with oxygen from an option oxygen cylinder 12 prior to reaching the patient. Additional features such as a detector or filters can also be included.

### Example 6: NO synthesis using ascorbic acid

The immediate treatment for patients with respiratory insufficiency and respiratory failure is provision of supplemental O₂ to improve oxygen saturation. However, supplemental O₂ is not sufficient if these symptoms are associated with certain types of pulmonary hypertension. Introducing a small amount of NO gas into the O₂ is one of the best options to improve the matching of ventilation to perfusion and dilate the pulmonary arteries to improve the blood flow and oxygen saturation. One major drawback to this treatment is that NO reacts with O₂ to generate toxic NO₂, which opposes the beneficial effect of NO. Hence the efficacy of inhaled NO treatment depends on the purity of the inhaled NO gas. The first major point of NO₂ production occurs during the synthesis of NO. To address this problem, the methods described herein can generate pure NO without need for further purification. The second major point of NO₂ production occurs when NO is introduced into the supplemental O₂. Most of the NO₂ forms at the mixing point, where the rate of NO reaction with O₂ is several times higher than that after dilution is completed. The instantaneous mixing procedure described herein can reduce the dilution time and reaction of NO with O₂ to generate NO₂.

Nitrite is known to protonate to nitrous acid (HNO₂) in weak acidic conditions. The pKa of HNO₂ is 3.4. In this pH region, HNO₂ also exists in the intermediate species of nitrosonium ion (HNO₂ ↔ NO⁺-H₂O), which can be reduced rapidly to NO by any electron donor compound such as ascorbic acid, polyphenols, etc. Herein ascorbic acid is used as a reducing agent to generate bulk amounts of NO that could be used as inhaled NO. NO⁺ also readily complexes with thiol residue molecules to form S-nitrosothiols. A low-molecular-weight thiol compound, N-acetylcysteine (NAC), is used to react with this NO⁺ to form S-nitrosoacetylcysteine (RSNO). This RSNO decomposes spontaneously to NO as a sustained source of NO or rapidly degrades by cuprous chloride (CuCl) to create a bulk source of NO.

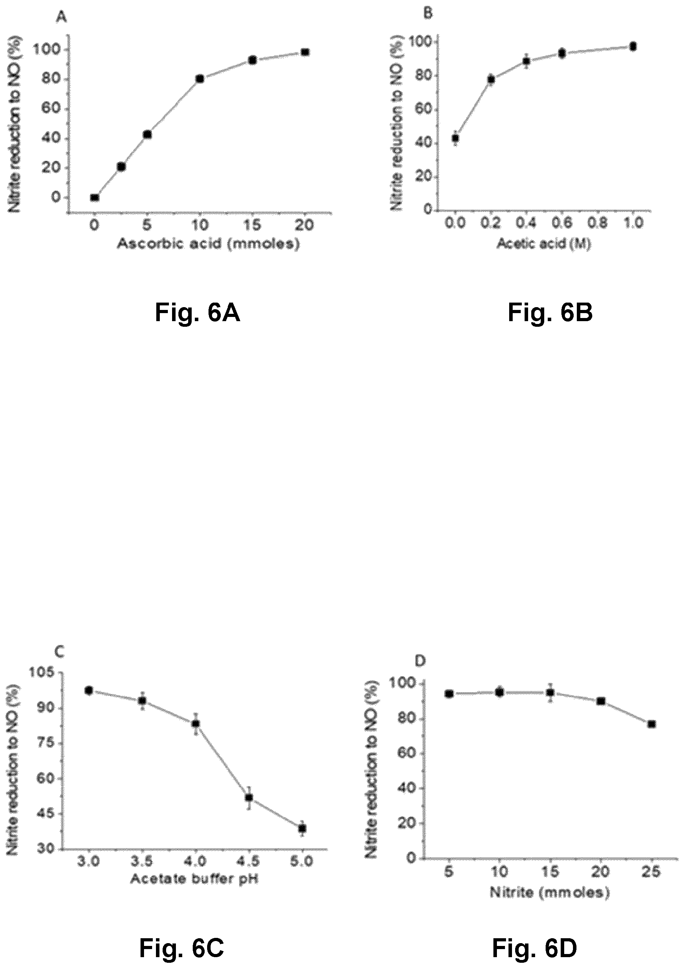

Figures 6A-6D illustrate NO synthesis by reduction of sodium nitrite with ascorbic acid or sodium ascorbate under acidic conditions. Three procedures have been used to synthesize NO from nitrite.

Conversion of nitrite to NO by ascorbic acid: Figure 6A shows the percent nitrite reduction in the presence of increasing concentrations of ascorbic acid in distilled water. Ascorbic acid (RH) ionizes to ascorbate anion and hydrogen cation (H⁺) in aqueous solutions (eq. 1). The pH of ascorbic acid at 0.25, 0.5, 0.75, and 1.0 M is 2.41, 2.34, 2.1, and 2.0 respectively. This pH is sufficient to acidify the nitrite to nitrous acid (HNO₂), which can exist as nitrosonium ion intermediate (NO⁺-H₂O) (eq. 2). Ascorbate anion can readily reduce NO⁺ ions to NO (eq. 3).

RH → R⁻ + H⁺ (eq. 1)

NO₂⁻ + H⁺ ↔ HNO₂ + H⁺ ↔ NO⁺-H₂O (eq. 2)

R⁻ + (NO⁺- H₂O) → NO + R' + H₂O (eq. 3)

According to ideal gas law, when 1 mole of mass substance converts completely to 1 mole of gas, it occupies 22.4 L at standard temperature (0°C) and standard atmospheric pressure (atm) (760 mm/Hg). A fixed quantity of 5 mmol of nitrite is reacted with increasing quantities of ascorbic acid (i.e., 2.5, 5, 10, 15, and 20 mmol) in an anaerobic reaction vessel that is flushed with inert nitrogen gas (N₂) to produce nitrite-to-ascorbic acid ratios of 1:0.5, 1:1, 1:2, 1:3, and 1:4 in 20 ml of distilled water. If 5 mmol (345 mg) of nitrite is completely reduced to NO, it occupies 112 ml volume that is considered to be 100% reduction. The amount of gas generated in the limited space of the reaction vessel is measured by a gastight graduated Hamilton syringe. The syringe needle is inserted into the vessel through the septum seal before initiation of the reaction. Any NO gas produced over the 1.12 atm transfers to the syringe due to mechanical force of pressure. The volume of the gas in the syringe is corrected to 1 atm pressure. Calculating the percent reduction at each concentration of ascorbic acid showed that 80% of nitrite is converted to NO at the nitrite-to-ascorbic acid ratio of 1:2, and 94% of nitrite is converted to NO at the nitrite to ascorbic acid ratio of 1:3 (Figure 6A). More than three-fold excess ascorbic acid is required to reduce the NO. After the reaction is completed, the pH of the reaction mixtures is increased to 5.6, 4.6, 3.9, and 3.6 at the nitrite-to-ascorbic acid ratios of 1:1, 1:2, 1:3, and 1:4 respectively. Thus, acidity is insufficient at nitrite-to-ascorbic acid ratios of less than 1:3.

Conversion of nitrite to NO by ascorbic acid in presence of an additional acid: Figure 6B shows the conversion of nitrite to NO by ascorbic acid in the presence of additional acid in the reaction mixture. The reduction of nitrite (5 mmol) to NO by an equal amount of ascorbic acid was investigated in the presence of varying concentrations of acetic acid from 0 to 1 M. Approximately 42% of nitrite was reduced to NO in the absence of acetic acid. This reduction was increased by increasing the acid concentration to 100% at 1 M acetic acid. Ascorbic acid reduces nitrite to NO at the stoichiometric ratio at this concentration of acid.

Conversion of nitrite to NO in acetate buffer media: Figure 6C illustrates conversion of nitrite to NO in acetate buffer media. The pH fluctuated greatly in the reactions described above owing to a lack of buffering capacity. pH fluctuations were also a major hindrance to generating more NO gas in a small volume of reaction mixture. Hence, an acetate buffer that has high buffering capacity in weak acidic conditions was used as reaction media. Fixed concentrations of 5 mmol nitrite and 5 mmol ascorbic acid were reacted in 1 M acetate buffer at varying pH: 3.0, 3.5, 4.0, 4.5, and 5.0. As shown in Figure 6C, 99% of nitrite was reduced to NO at pH 3.0, and 95% was reduced at pH 3.5. The 1 M acetate buffer at pH 3.5 was used for subsequent experiments.

Figure 6D shows that the maximum amount of nitrite is reduced to NO in 1 M acetate buffer at pH 3.5. We investigated the generation of NO in the presence of increasing concentrations of nitrite and ascorbic acid while keeping the ratio of 1:1 at a fixed concentration of 1 M acetate buffer. Approximately 94% of 20 mmol of nitrite was reduced to 428 ml of NO in the presence of an equal concentration of ascorbic acid and 1 M acetate buffer at pH 3.5.

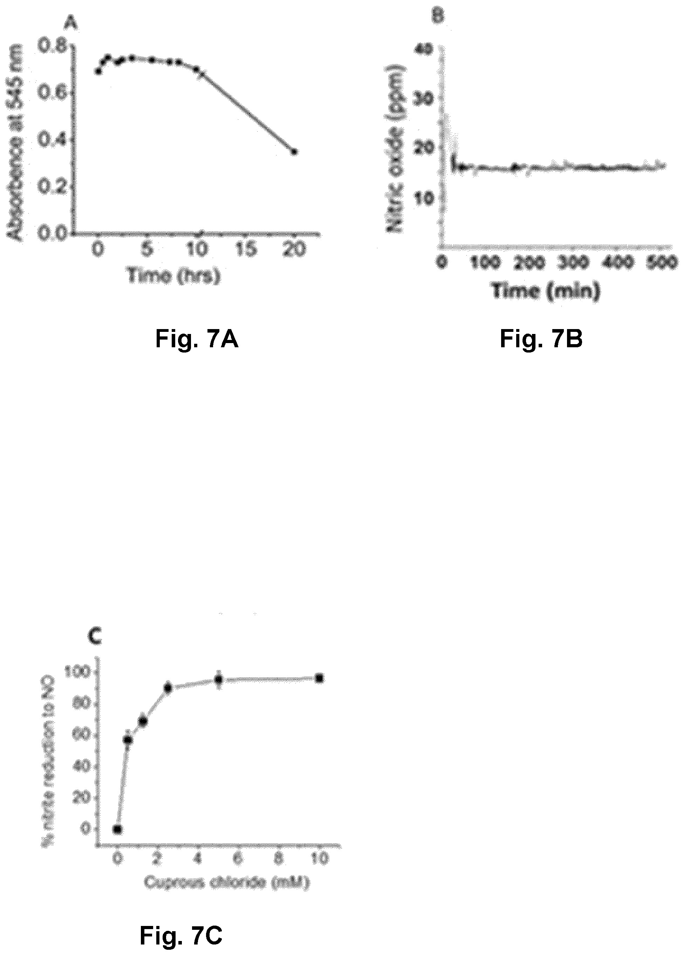

S-nitrosothiols as a source of NO (Figures 7A-7C): *N*-acetylcysteine was used to react with nitrite instead of ascorbic acid under acidic conditions to form S-nitrosoacetylcysteine (SNOAC). In contrast to ascorbic acid, the thiol group of cysteine complexes with NO⁺ ion to form SNOAC. The advantage of SNOAC is that it slowly decomposes to NO and disulfide. This NO is removed from the reaction vessel for use as inhaled NO. This SNOAC also can be used as a bulk source of NO when it is cleaved homolytically with metal catalyzed reactions.

### Example 7: NO synthesis using ascorbic acid N-acetylcysteine (NAC)

S-nitrosothiols as a source of NO (Figures 7A-7C): *N*-acetylcysteine was used to react with nitrite instead of ascorbic acid under acidic conditions to form S-nitrosoacetylcysteine (SNOAC). In contrast to ascorbic acid, the thiol group of cysteine complexes with NO⁺ ion to form SNOAC. The advantage of SNOAC is that it slowly decomposes to NO and disulfide. This NO is removed from the reaction vessel for use as inhaled NO. This SNOAC also can be used as a bulk source of NO when it is cleaved homolytically with metal catalyzed reactions.

Figure 7A shows the generation and decomposition of SNOAC. Aqueous *N-*acetylcysteine solution (0.5 M) was deoxygenated in a septum-sealed anaerobic vessel. Then 0.625 M nitrite was added and SNOAC generation and decomposition were measured spectrophotometrically over time at an absorption wavelength of 540 nm. Approximately 90% of nitrite was converted to SNOAC, which maintained a steady state level for 8 hours and then slowly decomposed to 50% in 20 hours.

Figure 7B shows the release of NO from SNOAC. In the presence of O₂, the NO released as SNOAC decays is oxidized to nitrite. However, in anaerobic conditions, it remains in the reaction vessel. This NO is extracted from the reaction vessel by a vacuum pump and then introduced into the air stream and determined by chemiluminescence. The NO release followed a trend similar to that of SNOAC decomposition (Figure 7A). The relatively weak S-N bond (bond dissociation energies are 20 to 32 kcal/mol) decays spontaneously to RS^{•} and NO^{•}. Hence, RSNO possesses a special ability to hold and release nitric oxide.

Figure 7C depicts bulk NO gas generation from SNOAC: Cuprous chloride (CuCl) rapidly cleaves the RS-NO bond homolytically to RS⁻ anion and NO (eq. 5). CuCl is less soluble in aqueous solutions and also unstable in oxygenated conditions. Hence more soluble and stable cupric chloride (CuCl₂) is reduced to CuCl by ascorbic acid (RH) (eq. 4) to degrade the RSNO to NO (eq. 4 to 7).

Cu(II) + RH → Cu(I) + R• (eq. 4)

RSNO + Cu(I) → RS⁻ + NO + Cu(ll) (eq. 5)

Cu(II) + RS⁻ → Cu(I) + RS^{•} (eq. 6)

RS' + RSNO → RSSR + NO (eq. 7)

Five mmol of nitrite was reacted with equal amounts of N-acetylcysteine in 20 ml of deoxygenated distilled water for 5 minutes to generate SNOAC in a septum-sealed anaerobic reaction vessel. In these conditions, 95% of nitrite was converted to SNOAC. This SNOAC was treated with various concentrations of CuCl₂ + ascorbic acid from 0.02 to 0.2 mmol. Then, the NO released into the reaction vessel headspace was immediately measured by syringe. This release of NO from RSNO increased with increasing concentrations of Cu(I) ions, and 100% of the 5 mmol of RSNO was completely converted to NO by 0.1 mmol of Cu(I). The IC50 for Cu(I) to decompose RSNO was 0.018 mmol under these conditions. The Cu(II) ions undergo redox reactions with RS⁻ to regenerate Cu(I) to decompose RSNO even at small concentrations (eq. 4-7).

### Example 8: Measurement of nitrogen dioxide

*Nitrogen dioxide contamination:* NO rapidly reacts with O₂ to generate NO₂, which is toxic to lungs. NO₂ is also an air pollutant, particularly in urban areas. US environmental agencies set a standard limit for NO₂ at 0.053 ppm. Workplace exposure limits for short time periods (15 min) are set at < 5 ppm by OSHA. The National Institute for Occupational Safety and Health (NIOSH) has set an airborne exposure limit at <1 ppm (NIOSH 2017). Hence, it is highly recommended that NO₂ remain <1 ppm for inhaled NO therapy. Air or oxygen is used as the carrier for small therapeutic doses of NO into the pulmonary system. Therefore, it is not possible to deliver the NO to patients without co-delivery of NO₂. However, it is possible to minimize NO₂ to safe levels. NO₂ generation in the current proposed inhaled NO can occur at several steps such as during synthesis, while mixing NO with oxygen, relative concentrations of these NO and O₂, and residence time of therapeutic gas (NO+ O2) in breathing circuit.

### Methods to determine NO:

*Chemiluminescence method:* Chemiluminescence measures NO, but not NO₂. For NO₂ concentration to be measured, it is first reduced to NO and then the total NO is determined. The NO₂ value is obtained by subtracting NO from total NO (see detailed explanation below).

NO₂ is soluble in aqueous solutions and can undergo several reactions with water, NO, and ascorbic acid to be converted to NO (eq. 8 to 11)

NO₂ + H₂O → HNO₂ + HNO₃ (eq. 8)

HNO₂ + RH → NO + R^{•} + H₂O (eq. 9)

2NO₂+2NO → 2N₂O₃ + RH + H⁺ → 4NO + R^{•} + 2H₂O (eq. 10)

NO₂ + RH → NO + H₂O + R^{•} (eq. 11)

Calibrated NO₂ gas was bubbled through the solution of 10% ascorbic acid in 50% glacial acetic acid. NO₂ was stoichiometrically converted to NO in aqueous ascorbic acid solution. The results suggested that NO₂ is probably directly converted to NO by forming N₂O₃ (eq. 10) and/or ascorbic acid (eq. 11). To determine NO and NO₂, we split the NO carrying gas line from the mixing chamber into two gas stream lines. One gas stream line is bubbled through the ascorbic acid-acetic acid solution for measurement of total NO₂ and NO. Another gas line is fed directly into the analyzer to measure only NO. The difference between these two values provides NO₂ levels.

*Electrochemical method (ECD):* In this method, an NO₂ sensor is used to determine the NO₂. NO₂ is determined by ECD at the same time as NO is determined by chemiluminescence. The NO₂ sensor is calibrated regularly with 5 ppm calibrated NO₂ gas. NO has some cross sensitivity with the NO₂ sensor, especially at higher concentrations. To determine NO and NO₂, we split the NO carrier gas line into two gas stream lines. One line is used to determine the NO₂ directly. Another gas line is bubbled through the ascorbic acid-acetic acid solution to convert the NO₂ to NO. Then the NO cross-reactivity is determined. The difference of these two values provides the true value of NO₂.

*Attenuated Phase Shift Spectroscopy (CAPS) NO₂ method:* CAPS NO₂ monitor (aerodyne) determines NO₂ by directly measuring optical absorption of NO₂ at 450 nm in the blue region of electromagnetic spectrum. This instrument is a gold standard to determine the NO₂ with the high sensitivity (< 1ppb) and without interference of other nitrogen species.

*NO₂ formation before injection into the mixing chamber:* For the device depicted in Figure 5A, the NO-generating reaction vessel (1 to 9), NO reservoir (12), and NO gas lines (10 to 19) are closed with airtight connections. The air in this system is removed by a mini-vacuum diaphragm pump (18) for 5 minutes. This step prevents any opportunity for NO₂ to be generated during synthesis or storage or during transfer to the mixing chamber if the system (1 to 19) is free from air or air leakage. The NO₂ formation in these systems is tested by mixing NO with nitrogen (inert gas) as a carrier gas in the mixing chamber. Although the chemiluminescence method is highly sensitive and provides a rapid response, the standard error was more than 1 ppm. Hence, we did not consider the chemiluminescence method for measuring NO₂ levels below 1 ppm. The NO₂ sensor method detected the presence of approximately 0.25 ppm NO₂ in 20 ppm of NO. This NO₂ is formed as a result of residual air in the vessel, as the vacuum pump does not remove all of the preexisting air. However, it has been observed that the initial level of NO₂ (0.25 ppm) decreases with time and reaches zero by 10 minutes. This decrease occurs because the water-soluble NO₂ diffuses from the gas phase to the liquid phase reaction mixture, where it is converted to NO by ascorbic acid (eq. 8-11). The complete conversion of gas phase NO₂ to NO by liquid phase ascorbic acid was further confirmed by injecting a large excess of NO₂ into the reaction vessel. For efficient conversion of NO₂ to NO, initially, a small amount of nitrite is allowed to convert to NO (20 ml). This NO rapidly reacts with any residual O₂ and forms NO₂. This less diluted NO₂ can diffuse quickly into the liquid phase and convert to NO in less than 5 minutes. Acidic ascorbic acid not only reduces nitrite to NO, but also reduces toxic NO₂ to NO. That is how pure NO gas is prepared in the reaction vessel.

*NO₂ levels in therapeutic gas (NO* + *O₂)*. As discussed above, most of the NO₂ is formed from the reaction of NO with O₂ at the mixing point before the dilution is completed. Therefore, the system includes a mixing method that dilutes NO with O₂ to therapeutic levels instantaneously to minimize NO₂ generation.

Tables 2 and 3 show the NO₂ levels detected by electrochemical sensor and CAPS 2 respectively. The NO₂ formation is dependent on the concentration of NO, oxygen, time to complete dilution in the mixing chamber, and resident time of NO and O₂ mixture in the breathing circuit.

**Table 2. NO₂ levels during mixing of NO gas with carrier gases detected by electrochemical sensors. The values are mean ± SD. N= 5 individual experiments.**

| Gas type | Gas flow rate | NO (20ppm) | NO (40ppm) | NO (80ppm) |
|---|---|---|---|---|
| Nitrogen | 2L/min | <0.1 | <0.1 | <0.1 |
| Air | 2L/min | 0.13 ± 0.016 | 0.22 ± 0.017 | 0.55 ± 0.028 |
| Oxygen | 2l/min | 0.32 ± 0.025 | 0.64 ± 0.031 | 1.85± 0.056 |
| Nitrogen | 4L/min | <0.1 | <0.1 | <0.1 |
| Air | 4L/min | 0.11 ± 0.015 | 0.18 ± 0.013 | 0.48± 0.032 |
| Oxygen | 4L/min | 0.30 ± 0.021 | 0.59± 0.05 | 1.75± 0.065 |

**Table 3. NO₂ levels during mixing of NO gas with carrier gases detected by CAPS 2. The values are mean ± SD. N= 5 individual experiments.**

| Gas type | Gas flow rate | NO (20ppm) | NO (40ppm) | NO (80ppm) |
|---|---|---|---|---|
| Nitrogen | 2L/min | <0.01± 0.001 | <.0.018± 0.004 | < 0.0 42± 0.01 |
| Air | 2L/min | 0.125 ± 0.018 | 0.25 ± 0.027 | 0.65 ± 0.048 |
| Oxygen | 2l/min | 0.35 ± 0.025 | 0.68 ± 0.035 | 2.05± 0.076 |
| Nitrogen | 4L/min | <0. 0.18 ± 0.001 | <0.039 ± 0.002 | < 0.082± 0.009 |
| Air | 4L/min | 0.122 ± 0.025 | 0.26 ± 0.018 | 0.62± 0.038 |
| Oxygen | 4L/min | 0.34 ± 0.021 | 0.61± 0.058 | 1.85± 0.085 |

NO concentrations of 20, 40, and 80 ppm in carrier gases of nitrogen (0% O₂), air (20% O₂), and 100% O₂ were investigated. Instantaneous mixing of NO with O₂ was achieved by using a nozzle system to inject NO at high flow velocity into passing O₂ that is also moving at high velocity. Because the gases mix together at high velocity, dilution time is decreased, thereby reducing the NO₂ formation. The resident time of O₂ + NO after exiting from the mixing chamber is dependent on the flow velocity in the breathing tube. This flow velocity is calculated based on the tubing internal diameter of 0.0625 inches and flow rate of 2, 4, and 6 L/min. The calculated resident time of gas for a 40-inch length of tubing from the mixing chamber to the facemask would be 0.06, 0.03, and 0.02 seconds for 2, 4, and 6 ml/min flow rate, respectively. Based on the kinetic rates, 80 ppm NO reacts with 100% oxygen to generate 9.4, 4.7, and 3.13 PPB at flow rates of 2, 4, and 6 L/min, respectively. Consequently, NO₂ generation during the resident time of NO in air or O₂ can be ignored, as these values are negligible. The NO₂ levels are less than 0.05 ppm in 80 ppm NO in nitrogen gas, indicating that this much amount of NO₂ is formed during the synthesis of NO. This NO does not need any further purification. In fact, this reaction system not only generates NO gas but also removes any traces of NO₂ generated. The NO₂ levels were 0.32 ppm, 0.62 ppm, and 1.85 ppm at concentrations of 20 ppm, 40 ppm, and 80 ppm NO, respectively when the oxygen flow rate was 2 L/min. Most of this NO₂ is likely generated at the mixing point of NO with O₂, as the NO₂ formation during the gas transit time is negligible. The rate of NO₂ formation is square to the NO concentration. In other words, every two-fold increase in NO concentration should result in a four-fold increase in NO₂ generation. This principle does not follow in the present experiments, however. In the present examples, increasing NO concentration from 20 ppm to 40 ppm and 80 ppm generates only two-fold and four-fold NO₂, respectively, rather than four-fold and 16-fold. The possible explanation for this result is that NO is delivered at the fixed orifice of the nozzle. As the NO injecting volume increases, the flow velocity of NO gas that is emerging from the nozzle's orifice also increases, thereby resulting in more rapid mixing with O₂. The more rapid mixing in turn decreases the dilution time, thereby decreasing NO reaction with O₂ to generate NO₂. The kinetic rates at which NO reacts with O₂ were established at fixed concentrations and in static conditions. This theory does not apply to gases that are moving at high velocity or when gas concentrations are changing. Hence, it is possible to administer the large concentrations of inhaled NO that are needed to treat pulmonary infections using the presently described delivery systems.

Current NO delivery systems on the market that use compressed tanks (800 ppm in N₂) are mostly limited to administration of 20 ppm because the NO₂ levels are reported to be 0.7 ppm. Some reports have even shown that at times, NO₂ generation reaches 3 ppm at the 20 ppm dose. Recently, the FDA approved a non-compressed gas delivery system, which is also limited to a 20 ppm dose level despite its use of NO₂ scavenger filters. The system described herein, which uses 100% NO without prior dilution in inert gas or air, can deliver NO at up to 40 ppm while generating less than 1 ppm NO₂, but does not require NO₂ filters or a scrubber system. Thus, the present NO generation and delivery system may provide economical and feasibility advantages over other systems on the market.

### Example 9: RSNO as a source for NO

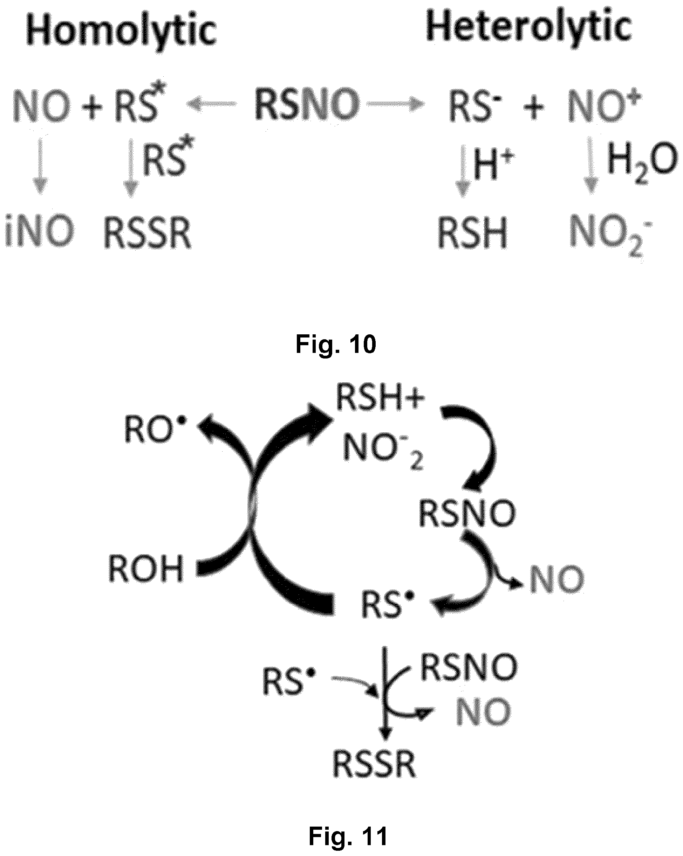

Carefully considering all the pros and cons of present systems, it is possible to develop portable NO-generating systems that are simple, inexpensive and produce desired levels of pure NO directly for the patients without need for purification. For this purpose, in the example disclosed herein, RSNO was chosen as a source of NO. RSNO was synthesized by reacting low molecular weight thiols with acidified nitrite, as discussed above. The relatively weak S-N bond (bond dissociation energy is ~ 20 to 32 cal /mole and bond length ~1.8 A⁰) spontaneously decays via homolytic cleavage to generate RS^{•} and NO^{•} or heterolytic cleavage to RS⁻ and NO⁺ (these species convert back to RSH and nitrite following reaction with protons and water, respectively. Less energy is required for homolytic cleavage compared to heterolytic cleavage (Figure 10). RSNO possesses a special ability to store and release of nitric oxide.

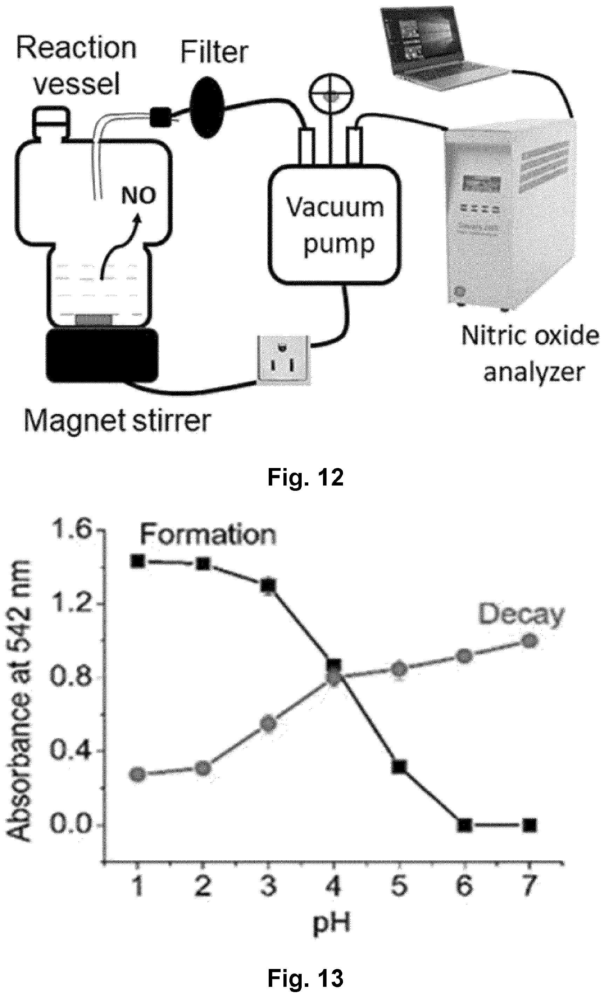

Reaction Vessel: A vessel was designed to perform the chemical reactions to generate NO under anaerobic conditions and from which the NO can be extracted (Figure 12 12). A prototype glass reaction vessel was built with a 30 mL to 50 mL capacity. The device has a sample inlet port and screw cap with rubber septum, gas evacuation line. The gas evacuation line is designed in such a way that the solution does not interfere with the line even when the vessel is tilted to any direction or upside down. A magnetic bar in the cell stirs the solution at a constant rate using a magnetic stirrer to diffuse the NO uniformly into the cell's headspace (Figure 12).

Removal of NO gas from the reaction vessel. A mini-vacuum pump (KNF N86KT-46P) is used to draw the NO from the reaction vessel. The vacuum pump has a regulator to adjust the gas flow rate from 0.5 to 5L/min. The gas outlet line is connected to a Sievers nitric oxide analyzer to monitor the NO release (Figure 12) and exhaust hood.

Choosing RSNO compound as source of NO: Low molecular weight thiols such as cysteine, N-acetylcysteine and glutathione were reacted with acidified nitrite to generate corresponding S-nitrosocysteine, S-nitrosoacetylcysteine, and S-nitrosoglutathione. It was found that S-nitrosoacetylcysteine is suitable as a source for NO, based on its stability, cost and availability. For simplicity, in this example, *N*-acetylcysteine and S-nitrosoacetylycysteine are abbreviated as RSH and RSNO respectively. Other thiols could be used, as can be envisioned by one of skill in the art.

Methods: All the reactions were conducted in the presence of 0.5 M EDTA at room temperature and protected from light. The absorption spectrum was measured from 700 to 400 nm by Lambda 35 UV/Vis spectrophotometer (PerkinElmer). The concentration of RSNO was determined by using the extinction coefficient of 16 M⁻¹ cm⁻¹ at 545 nm.

*Nitric oxide measurement:* Nitric oxide was measured by chemiluminescence using Sievers Nitric Oxide Analyzer (NOA), model 280.

*Finding the right pH for generation and decomposition of RSNO:* The rate kinetics reported in the literature for nitrite's reaction with RSH to generate and break down RSNO could not be applied to the experiments described herein because they were conducted under anaerobic conditions and NO was removed from the reaction mixture. In the reaction vessel, equal ratios of nitrite (0.1 M) and RSH (0.1 M) were reacted in 2 M sodium acetate buffer containing 0.5 mM EDTA at pHs that varied from 7 to 1 as NO was removed by the vacuum system. At various time points over an hour, 0.2 mL of reaction mixture was extracted by gastight syringes, and RSNO formation was measured (Figure 13).

*Results.* As shown in Figure13, the rate and amount of RSNO formation increased with decreasing pH and reached a maximum by pH 2.0. Although the pKa of HNO₂ is 3.4, 60% of RSH was converted to SNOAC at pH 4.0 under these conditions (Figure 13).

*Effect of pH on the release of NO from RSNO.* RSNO (0.1M) was generated at pH 1 then adjusted to 2 or 3 or 4 or 5 or 6 or 7. The decay of RSNO and release of NO were monitored at each pH level for 10 h. The decay decreased with decreasing the pH (Figure 13).

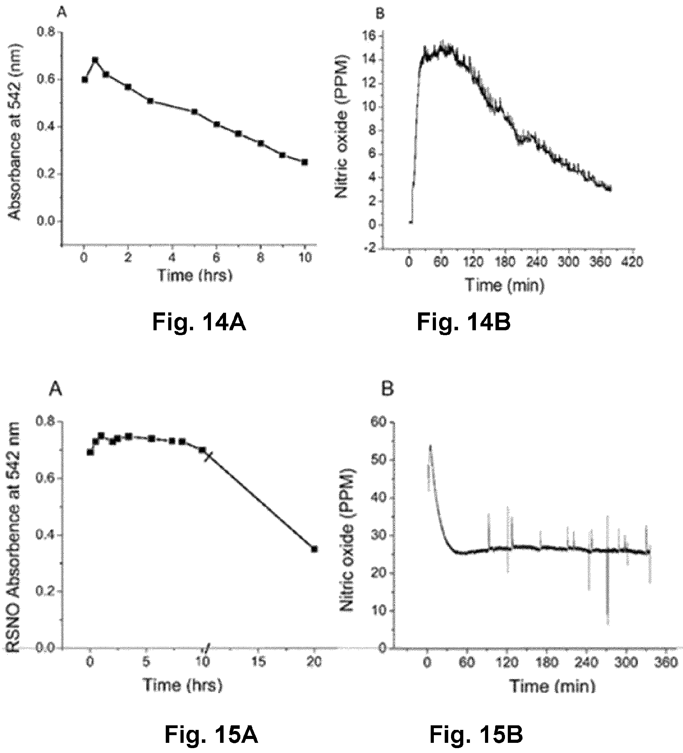

*NO release during the reaction of RSH with nitrite:* NAC was reacted with nitrite in 0.25 M HCl to generate RSNO. Then RSNO decay (Figure 14A) and NO release (Figure 14B) were measured. SRSNO generation was rapid initially, plateaued for 45 min, and then slowly waned with time. The release of NO followed the same trend.

*Sustained release of NO for more than 1 hr.* Above experiments indicate that the decomposition of RSNO is correlated with the release of NO. Therefore, RSNO levels need to be maintained to release the sustained levels of NO (*i.e*. to extend the plateau region in Figure 14B. Based on the above results, pH 2 was used for generation of RSNO and pH 4.5 for decomposition of RSNO to yield NO. The reason for choosing pH 4.5 is that RSNO generates (Figure 13) and decomposes slowly at this pH. Nitrite and RSH were reacted 1:1 ratio in water to generate RSNO. About 90% of nitrite was converted to RSNO and maintained a steady state level for 6 hrs and then slowly weaned off to 80% in 15 hrs (Figure 15A). The unreacted nitrite reacts slowly with RSH at less acidic pH of 4.5 to generate RSNO to compensate for the decaying RSNO. NO release also followed the same trend (Figure 15B).

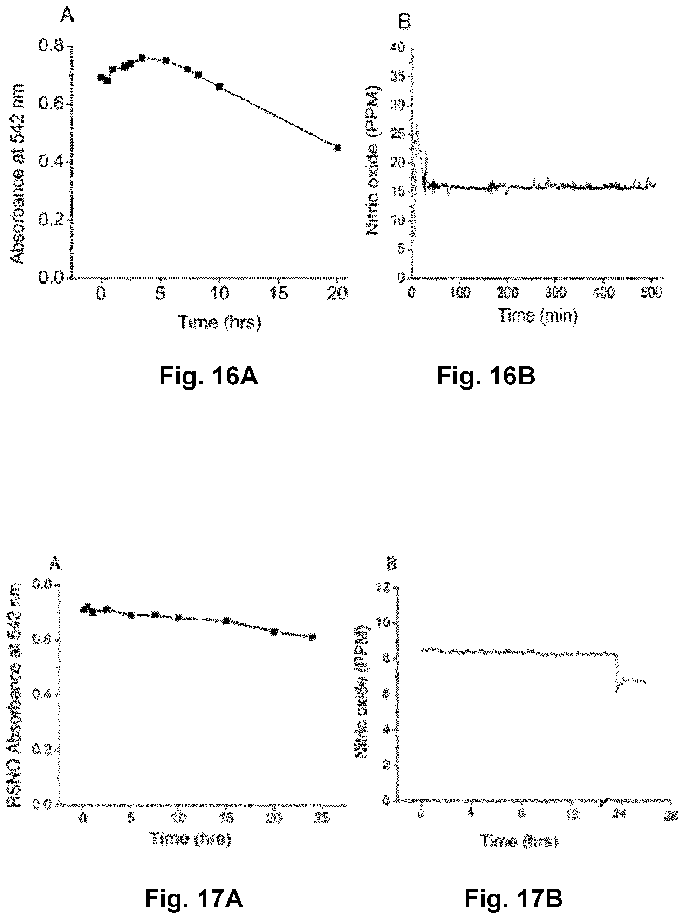

*Sustained release of NO for more than 6 hrs.* Herein, pH 2.75 was used for generation of RSNO and pH 4.8 for decomposition of RSNO to yield NO. The pH of the 0.5 M RSH was adjusted to 2.75 with alkali and then reacted with 0.625 M nitrite to generate RSNO. The pH of this reaction mixture was raised to 4.8 because nitrite in water is base. About 90% of nitrite was converted to RSNO, which maintained a steady state level for 10 h and then slowly decreased to 80% in 15 h (Figure 16A). The unreacted nitrite reacts slowly with RSH at the less acidic pH of 4.8 to generate RSNO to compensate for the decaying RSNO. NO release followed a similar trend to that of RSNO decay (Figure 16B).

*Sustained release of NO beyond 10 h.* To further extend the NO release, RS* radicals were reduced to RSH to participate in another reaction with nitrite. This process allowed RSNO to be maintained at steady state levels to yield NO. The phenolic antioxidant quercetin (ROH) was used to scavenge RS* radical and reduce to RSH (Figure 11). This RSH can participate in another round of reactions with nitrite to generate RSNO, thus maintaining steady state levels of RSNO to yield NO. RSH (0.5 M) was reacted with 1 M nitrite in the presence of 50 mM quercetin. The pH of the final reaction mixture was 4.2. The steady state level of RSNO (SNOAC) was maintained for 15 h and had declined by approximately 20% at 24 h (Figure 17A-17B). The NO release followed a trend similar to that of RSNO. The amount of NO release was 50% less than it was in the previous experiment (Figure 16B) because the antioxidant prevented RSH* reaction with RSNO to release more NO (Figure 11).

*Conclusion:* In the studies described above, the decay of RSNO and NO release was extended from 1 hour to at least 15 hours.

One goal of the studies of the present disclosure is to develop portable and benchtop drug-devices to generate medical NO gas by a simple chemical method to use in and out of hospitals by using the NO that releases from S-nitrosothiols in aqueous solution as a source for iNO therapy. The studies described herein show that S-nitrosoacetylcysteine, prepared by reacting nitrite with N-acetylcysteine, is an ideal compound for this purpose. An initial problem was that the rate of S-nitrosoacetylcysteine decomposition to yield NO is faster than that of time required for iNO therapy. Hence the reaction conditions have been optimized in such a way to allow sustained and stable release of NO for extended periods of time. These studies establish the amount of nitrite, RSH and reaction conditions required to generate the therapeutic levels of NO for a desired length of time. These chemicals may be formulated in a tablet form, a powder form, or the like, to use readily.

Studies such as those described above have established that S-nitrosoacetylcysteine (RSNO) generated by reaction of nitrite with N-acetylcysteine (RSH) is an ideal NO donor compound. It has also been shown herein that manipulating the reaction conditions, especially pH, but also neutralizing RS radicals, proper ratio of nitrite to RSH, absence of oxygen, metal chelators and subdued light could prolong the rate of RSNO decomposition to yield NO.

### Example 10: Methods for weaning off NO generation for treatment:

Wean off NO (5, 10 and 20ppm) generation to use for acute and chronic pulmonary arterial hypertension (PAH) treatment: PAH is highly debilitating disease. A combination of drugs that are involved in nitric oxide, prostacyclin and endothelin pathways have been suggested to be more effective in managing PAH than single drug treatment. Addition of iNO to these drugs could improve the quality of life and survival rate. Continuous iNO therapy has been shown to have some side effects like down regulation of endogenous NO synthesis and/or elevated endothelin-1 and rebounding of the PAH more than basal level upon sudden withdrawal of the treatment. Even small levels of NO₂ contaminant can cause toxicity to the already sick lungs, although Occupational Safety and Health Administration sets a permissible NO₂ exposure limit to 5 ppm for the general population. These side effects can be minimized by administering weaning NO as shown in Figures 14A-14B. It is believed that this strategy of iNO therapy can improve the systemic oxygenation without side effects.

*Procedure:* The AC powered magnetic stirrer and the vacuum pump (FIG. 12) described above can be replaced with a battery powered mini-magnetic stirrer and diaphragm pump (FIG. 18). The same components of reaction vessel, magnetic stirrer and diaphragm pump can be used to build an instrument for phase 2 studies. All reactions will be conducted in the presence of EDTA to avoid metal contamination and subdued light. Higher ratio of RSH to nitrite and low pH (1 to 2) without RS^{•} scavenger will be used for weaning NO in 8 hours. EDTA (0.5mM) contained distilled water (10ml) will be taken in the reaction vessel and deoxygenated by vacuum system. Powder form of 492mg RSH (300 mM) and 100mg nitrite (150 mM) will be mixed together and then introduced immediately into the cell through the sample port and seal it. RSNO generates instantly and releases NO. NO release and NO₂ formation can be monitored continuously using chemiluminescence and electrochemical detector respectively. The ratio of RSH to nitrite and pH will be changed to extend or reduce the duration of NO weaning based on the initial results. For example, to extend the weaning period, the RSH concentration will be reduced and pH will be increased. To retract the weaning time, the RSH concentration will be increased and pH will be decreased. Excess free thiols and low pH increases decomposition of RSNO. If necessary phosphoric acid will be added to further reduce the pH. Once the ratio of RSH to nitrite is established for the desired weaning period, the amount of RSH and nitrite will be increased or decreased at the same ratio to obtain the desired levels of NO. The amount of NO release is proportional to the steady state levels of RSNO at the constant reaction conditions. Exact concentrations of RSH and nitrite and reaction conditions will be established for generation of 5, 10 and 20 ppm NO in 1L or 2L/min air flow. NO₂ formation will also be measured along with NO. Once the system is established for consistent generation of NO within acceptable error, the monitoring devices for NO, NO₂ and O₂ may not be required. These devices may be used for treatment of acute and chronic conditions of PH associated diseases of PAH, COPD and ARDS, sleep apnea and other lung diseases while patients are in rest or walking or sleep. Battery operated portable devices may be used for daytime and AC powered non-portable device may be used at night times.

Sustained release of NO for 6 hrs. to use in emergency conditions. iNO therapy has been used for the management of life-threatening PH (acute right heart failure, acute lung injury/ARDS, COPD and acute sickle heart crisis. This therapy is not available in emergency situations unless and until the patient reaches to ICUs of major hospitals. Meanwhile there is a possibility of developing irreversible organ damage, particularly to the brain, or even death. Therefore, the development of a ready-to-use, portable iNO therapy device to bridge the gap between the onset of emergency situation and rescue therapy at ICU is important.

*Method:* This method is also similar to that of above wean off NO except equal molar ratios of RSH and nitrite or less ratio of RSH to nitrite (1 to 1.25) will be used. For example, equal molar concentration of 1g RSH (612 mM) and 424 mg (612 mM) nitrite can be mixed and add to 10ml distilled water without acid and monitor the NO release and NO₂ formation. If this ratio does not release NO continuously, the nitrite level may be increased compared with RSH and the NO release checked. The reaction may also be performed in acetate buffer (2 M to 4 M) in between pH 4 to 4.9 where the nitrite conversion to NO is slow as well as decomposition of RSNO is also slow. The chemical composition required will be determined for generation of the exact amounts of 10 ppm, 20 ppm, and 40 ppm NO for 6 hours. NO₂ levels may be higher in the beginning minutes. The inhalation of NO will be initiated after reaching the safety levels of NO₂. The design of the reaction vessel may enable the chemicals to be dropped into solution anaerobically.

Sustained release of NO for 24hrs and beyond. The RS radical formed after NO is released from RSNO, converting to the irreversible product of RSSR. In order to prevent this RSSR formation, the RS radical is reduced to RSH by a free radical scavenger (e.g. the phenolic antioxidant), so that this RSH can react with nitrite again to generate RSNO to maintain RSNO levels in spite of decaying to NO. Nitrite is reduced to NO and removed from the reaction vessel. To compensate this loss, the ratio of nitrite to RSH is kept at 2:1. This nitrite exists as in the equilibrium of NO₂ ↔ HNO₂ until RSH is available to react. The amount of excess nitrite required will be calculated based on the NO in exited NO-air mixture and flow rate and duration of experiment. **Procedure:** The same reaction vessel and magnetic stirrer may be used for all experiments and all devices. The vacuum pump will be selected as appropriate, based on the use in a portable or non-portable device. The amount of nitrite and RSH required in the presence of an appropriate amount of free radicals scavenger to generate 2 ppm to 100 ppm in 10ml water will be further investigated. Based on the preliminary studies, the NO is generated continuously as long as nitrite and antioxidant are available. The ideal concentrations of RSH, nitrite and free radical scavenger required to generate continuously from about 24 hrs. to 3 days will be established. Physicians may wish to change the dose level based on the oxygenation of blood. A restrictor to control gas flow and / or measuring devices to monitor the gas concentrations may be provided. **Applications:** Continuous treatment may be necessary for stationary and non-stationary patients who are moving within hospitals for diagnostic tests or transfer to other hospitals or homes. These devices can be developed for use as an attachment (e.g. to wheelchairs, wheel stands, and bench tops). These devices may additionally have electro chemical devices for monitoring NO, O₂ and NO.

***Formulation of chemicals in a tablet form:*** After establishing the required chemical composition for each level of NO generation for sustained release, it may be possible to formulate these chemicals into a tablet form to use readily. Preliminary studies show that powder form of nitrite and RSH do not react in a moisture-free environment, but they do react in a moist environment. In the event that RSH and nitrite are not able formulate as a mixture, it may be possible to formulate these chemicals in layers separated by inert materials.

### Aspects of the Disclosure

The present disclosure will be better understood upon reading the following numbered aspects, which should not be confused with the claims. Any of the numbered aspects below can, in some instances, be combined with aspects described elsewhere in this disclosure and such combinations are intended to form part of the disclosure.

Aspect 1. A NO generating system comprising: a reaction vessel having a headspace for NO accumulation; a NO storage system; and a gas mixing chamber; wherein NO gas generated in the reaction vessel is delivered to the NO storage system via pressure generated by generation of the NO gas, then the NO gas is mixed with a carrier gas in a gas mixing chamber; and wherein the gas mixing chamber comprises a valve, a nozzle, a carrier gas inlet, and an outlet for a breathing tube, wherein the valve and nozzle control a flow rate of NO to the mixing chamber.

Aspect 2. The system of aspect 1, wherein the reaction vessel is the NO storage system, wherein the reaction vessel can withstand gas pressure higher than 1 atm, and wherein the pressurized NO gas is delivered from the reaction vessel to the mixing chamber at a desired flow rate via a gas regulator.

Aspect 3. The system of aspect 2, wherein the gas regulator has an inlet and an outlet, and wherein the gas regulator maintains a constant outlet pressure of 0 to 15 psi.

Aspect 4. The system of aspect 1, wherein the NO storage system comprises a gastight syringe, the syringe comprising: a plunger controlled by a drive shaft and motor; and wherein the NO reservoir is in fluid communication with the mixing chamber via a gas line, and wherein an infusion pump is in communication with a computer processing unit to control the flow rate of NO to the mixing chamber.

Aspect 5. The system of any of aspects 1-4, wherein the NO is sprayed via the nozzle into flowing carrier gas supplied through the carrier gas inlet to dilute the NO gas, and wherein the carrier gas is O₂.

Aspect 6. The system of aspect 5, wherein the NO gas is diluted to about 5 ppm to 80 ppm before a NO reaction with O₂ generates NO₂, and wherein the concentration of NO is about 1 million ppm to 20 ppm to obtain a therapeutic dose of about 20 ppm.

Aspect 7. The system of any of aspects 1-6, wherein reactants are reacted in an aqueous solution in the reaction vessel under anaerobic conditions.

Aspect 8. The system of any of aspects 1-7, further comprising a NO delivery system configured to deliver diluted NO to a patient in need thereof, wherein the NO delivery system is connected to an outlet of the gas mixing chamber by a breathing tube.

Aspect 9. The system of aspect 8, the breathing tube further comprising a gas flow sensor, and electrochemical detector, or a combination thereof.

Aspect 10. The system of aspect 9, wherein the electrochemical detector is configured to detect NO, O₂, NO₂, or a combination thereof.

Aspect 11. The system of any of aspects 1-10, further comprising an oxygen cylinder coupled to the carrier gas inlet.

Aspect 12. The system of any of aspects 1-11, further comprising a power source, a user input, a memory, and a CPU.

Aspect 13. A method for generating NO for inhaled therapy comprising: (a) reacting a nitrite and an electron donor compound in an aqueous solution, wherein the reaction is performed under anaerobic conditions at a pH of about 3 to 3.5; or (b) reacting a nitrite and a thiol (RSH) in an aqueous solution, wherein the reaction is performed under anaerobic conditions in the absence of light at a first pH of about 1 to 4.5, wherein the reaction produces RSNO and at least a portion of the RSNO decomposes to NO.

Aspect 14. The method of aspect 13, wherein the nitrite is selected from the group consisting of sodium nitrite, potassium nitrite calcium nitrite, zinc nitrite, copper nitrite, rubidium nitrite, strontium nitrite, and barium nitrite.

Aspect 15. The method of aspect 13, wherein the nitrite is NaNO₂.

Aspect 16. The method of aspect 13, wherein the electron donor compound is selected from the group consisting of ascorbic acid and polyphenols.

Aspect 17. The method of aspect 15, wherein the concentration of the electron donor compound is about 0.25 M to 1 M and the ratio of nitrite-to-electron donor compound is about 1:3 to 1:4.

Aspect 18. The method of aspect 13, wherein the step of reacting a nitrite and an electron donor compound is performed in the presence of acetic acid or acetate buffer.

Aspect 19. The method of aspect 13, wherein the RSH is selected from the group consisting of: cysteine, N-acetylcysteine, glutathione, mercaptoethylamine, mercaptopropionic acid, N- acetyl-dl-pencillamine, and Captopril.

Aspect 20. The method of aspect 13, wherein the RSNO is selected from the group consisting of S-nitrosocysteine, S-nitroso-N-acetylcysteine, S-nitrosoglutathione, S-nitrosomercaptoethylamine, S-nitrosopropionic acid, S-nitroso-N-acetylpenicillamine and S-nitrosocaptopril.

Aspect 21. The method of aspect 13, wherein the RSH is N-acetylcysteine, wherein the nitrite is NaNO₂, and wherein the RSNO is S-nitrosoacetylcysteine.

Aspect 22. The method of aspect 13, further comprising providing the RSNO with an amount of CuCl sufficient to degrade the RSNO to NO.

Aspect 23. The method of aspect 22, wherein the CuCl is formed by reacting CuCl₂ and ascorbic acid.

Aspect 24. A NO generating system comprising: a reaction vessel having a headspace for NO accumulation; a NO storage system; and a gas mixing chamber; wherein NO gas generated in the reaction vessel is delivered to the NO storage system via pressure generated by generation of the NO gas, then the NO gas is mixed with a carrier gas in the gas mixing chamber.

Aspect 25. The system of aspect 24, wherein the reaction vessel is the NO storage system, wherein the reaction vessel can withstand gas pressure higher than 1 atm, and wherein the pressurized NO gas is delivered from the reaction vessel to the mixing chamber at a desired flow rate via a gas regulator.

Aspect 26. The system of aspect 24, further comprising a gas regulator, wherein the gas regulator has an inlet and an outlet, and wherein the gas regulator maintains a constant outlet pressure of 0 to 15 psi.

Aspect 27. The system of aspect 24, wherein the NO storage system comprises a gastight syringe, the syringe comprising: a plunger controlled by a drive shaft and motor; and wherein the NO reservoir is in fluid communication with the mixing chamber via a gas line, and wherein a vacuum pump and at least one valve controls the flow rate of NO to the mixing chamber.

Aspect 28. The system of any of aspects 24-27, wherein the gas mixing chamber comprises: a valve and a nozzle to control a flow rate of NO to the mixing chamber; a carrier gas inlet; and an outlet for a breathing tube.

Aspect 29. The system of aspect 28, wherein the NO is sprayed via the nozzle into flowing carrier gas supplied through the carrier gas inlet to dilute the NO gas, and wherein the carrier gas is O₂.

Aspect 30. The system of aspect 29, wherein the NO gas is diluted to about 5 ppm to 80 ppm before a reaction of the NO with the O₂ generates NO₂.

Aspect 31. The system of aspect 24, wherein reactants are reacted in an aqueous solution in the reaction vessel under anaerobic conditions.

Aspect 32. The system of any of aspects 24-35, further comprising a NO delivery system configured to deliver diluted NO to a patient in need thereof, wherein the NO delivery system is connected to an outlet of the gas mixing chamber by a breathing tube.

Aspect 33. The system of aspect 32, wherein the breathing tube further comprises a gas flow sensor, an electrochemical detector, or a combination thereof.

Aspect 34. The system of aspect 33, wherein the electrochemical detector is configured to detect NO, O₂, NO₂, or a combination thereof.

Aspect 35. The system of any of aspects 24-34, further comprising an oxygen cylinder coupled to the carrier gas inlet.

Aspect 36. The system of any of aspects 24-35, further comprising a power source, a user input, a memory, and a CPU.

Aspect 37. A method of treating a patient in need of inhaled NO, comprising: reacting in a reaction vessel a nitrite and an electron donor compound in an aqueous solution, wherein the reaction is performed under anaerobic conditions at a pH of about 3 to 5 to form NO; removing the NO from the vessel to a NO storage system; diluting the NO to 80 ppm or less with oxygen in a mixing chamber; and providing, via a tube, the diluted NO to the patient for inhalation.

Aspect 38. The method of aspect 37, wherein the patient has at least one of a pulmonary arterial disease, a lung disease, sleep apnea, high altitude exposure, an infection, has a need for cardiac surgery, or is recovering from a cardiac surgery.

Aspect 39. The method of aspect 37, further comprising monitoring the removed NO for contaminants via a detector before the NO is provided to the patient.

Aspect 40. The system of aspect 5, wherein the nozzle has an inner nozzle orifice diameter between about 10 nm and about 100 nm.

Aspect 41. The system of aspect 40, wherein the nozzle has an inner nozzle orifice diameter selected from the group consisting of: about 10 nm, about 25 nm, and about 50 nm.

Aspect 42. The system of aspect 5, wherein the nozzle accelerates the NO gas to a velocity of about 0.1 m/s to 30 m/s.

Aspect 43. The method of aspect 13, wherein the RSNO is rapidly decomposed to NO by a metal catalyst to generate bulk amount of NO.

Aspect 44. The method of aspect 43, wherein the metal catalyst is cuprous chloride.

It should be noted that ratios, concentrations, amounts, and other numerical data may be expressed herein in a range format. It is to be understood that such a range format is used for convenience and brevity, and thus, should be interpreted in a flexible manner to include not only the numerical values explicitly recited as the limits of the range, but also to include all the individual numerical values or sub-ranges encompassed within that range as if each numerical value and sub-range is explicitly recited. To illustrate, a concentration range of "about 0.1% to about 5%" should be interpreted to include not only the explicitly recited concentration of about 0.1 wt. % to about 5 wt. %, but also include individual concentrations (e.g., 1%, 2%, 3%, and 4%) and the sub-ranges (e.g., 0.5%, 1.1%, 2.2%, 3.3%, and 4.4%) within the indicated range. In an embodiment, "about 0" can refer to 0, 0.001, 0.01, or 0.1. In an embodiment, the term "about" can include traditional rounding according to significant figures of the numerical value. In addition, the phrase "about 'x' to 'y'" includes "about 'x' to about 'y'''.

It should be emphasized that the above-described embodiments of the present disclosure are merely possible examples of implementations, and are set forth only for a clear understanding of the principles of the disclosure. Many variations and modifications may be made to the above-described embodiments of the disclosure without departing substantially from the spirit and principles of the disclosure. All such modifications and variations are intended to be included herein within the scope of this disclosure.

## Claims

1. A system comprising:
a nitric-oxide vessel (1) containing nitric oxide (2);
a mixing chamber (9);
a nozzle (10) coupled to the nitric-oxide vessel (1), the mixing chamber (9), and an oxygen source (12); and
an inspiratory tube coupled to the mixing chamber (9);
**characterized in that**:
the nozzle (10) has an inner nozzle orifice diameter between about 10 µm and about 50 µm; and
the nozzle (10) causes instantaneous mixing of the nitric oxide (2) with a carrier gas; and
the system does not include a nitrogen dioxide (NO₂) scrubber.

2. The system of claim 1, further comprising:
a gas regulator (3, 14) positioned between the nitric-oxide vessel (1) and the nozzle (10).

3. The system of claim 2, wherein the gas regulator (3, 14) is configured to maintain a constant outlet pressure of 0 kPa (0 psi) to 103 kPa (15 psi).

4. The system of any one of claims 1-3, wherein the inner nozzle orifice diameter is about 25 µm.

5. The system of any one of claims 1-3, wherein the nozzle (10) accelerates the pure nitric oxide (2) to a velocity of about 0.1 m/s to 30 m/s.

6. The system of any one of claims 1-5, wherein an output of the inspiratory tube contains:
NO at up to 40 ppm; and
less than 1 ppm NO₂.

7. The system of any one of claims 1-6, wherein the carrier gas is oxygen.

8. The system of any one of claims 1-6, wherein the carrier gas is air.

9. The system of any one of claims 1-8, further comprising:
a gas-monitoring device

10. The system of any one of claims 1-9, wherein the nitric-oxide vessel (1) contains pure nitric oxide (2).
